(19) Europäisches Patentamt
European Patent Office
Office européen des brevets

(11) Publication number: 0 018 305 A1

# (12) EUROPEAN PATENT APPLICATION

(21) Application number: 80710008.6

(22) Date of filing: 19.03.80

(51) Int. Cl.³: C 07 D 498/04, C 07 D 205/08, C 07 D 499/00, A 61 K 31/42 // (C07D498/04, 205/00, 263/00)

(30) Priority: 26.03.79 GB 7910487

(43) Date of publication of application: 29.10.80 Bulletin 80/22

(84) Designated Contracting States: AT BE CH DE FR GB IT NL

(71) Applicant: HOECHST U.K. LIMITED, Hoechst House Salisbury Road, Hounslow Middx. TW4 6JH (GB)

(72) Inventor: Ross, Barry Clive, Dr., 5 Upton Close, Luton Bedfordshire (GB)
Inventor: Johnson, Graham, Dr., 6 Golden Drive Eaglestone, Milton Keynes Buckinghamshire (GB)
Inventor: Yeomans, Michael Alan, Dr., 71 Shenley Road, Bletchley Buckinghamshire (GB)
Inventor: Cooke, Michael David, 10 Westbury Lane, Newport Pagnell Buckinghamshire (GB)

(74) Representative: Meyer-Dulheuer, Karl-Hermann, Dr. et al, HOECHST Aktiengesellschaft Zentrale Patentabteilung Postfach 80 03 20, D-6230 Frankfurt/Main 80 (DE)

## (54) Oxapenem derivatives, their preparation, their use, pharmaceutical compositions containing them, diverse initial compounds and their preparation.

(57) Disclosed is a process for the production of a compound of the general formula I

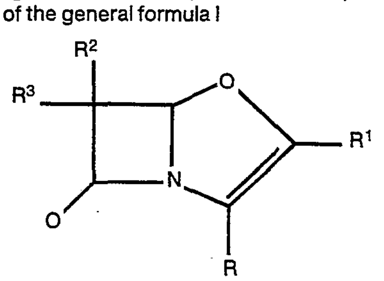

(I)

or a salt thereof, which comprises reacting a compound of the general formula II

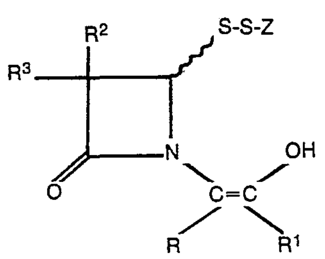

(II)

with a tervalent organophosphorus compound and, if desired, simultaneously or subsequently with a thiophilic agent as hereinbefore defined.

Compounds (I) and (II), pharmaceutical compositions containing compounds (I) and the use of these compounds as β-lactamase inhibitors and or as antibacterial agents are described.

Also desribed are initial compounds (penicillins, acetidinones) and processes for their preparation.

EP 0 018 305 A1

ACTORUM AG

BAD ORIGINAL

COMPLETE DOCUMENT

This invention relates to a process for producing oxapenem derivatives, to certain oxapenem derivatives, and to pharmaceutical preparations comprising oxapenem derivatives.

Processes are known for the production of 1-oxapenems. One process uses, as a starting material, clavulanic acid and is described in Belgian Patent Specification No. 858 515. Another process is described by, for example, P.H. Bentley, G.Brooks, M.L.Gilpin, and E.Hunt, J.Chem.Soc. Chem. Commun., (1977) 905. This procedure does not use a natural product as a starting material and involves a multistep procedure.

We have now found that certain 1-oxapenem derivatives can be readily prepared from 6-amino-penicillanic acid, a relatively inexpensive and readily available starting material. This process has the additional advantage that it allows the control of the stereo chemistry at positions 5 and 6 of the 1-oxapenem structure.

The term "oxapenem" is used herein to denote the structure

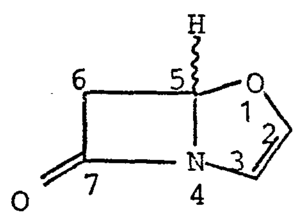

When the 6-position is substituted by 1 or more carbon atoms, the following numbering system is used herein

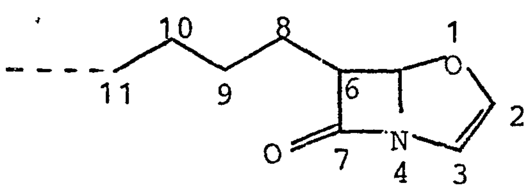

The numbering of the various positions is in accordance with that used in the chemistry of penicillins.

The present invention provides a process for the production of a compound of the general formula I

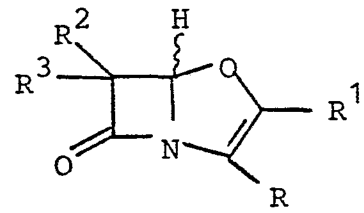

(I)

in which R represents a free or esterified carboxyl group,

$R^1$ represents a hydrogen atom or an unsubstituted or substituted straight or branched chain aliphatic group, and

$R^2$ and $R^3$, which may be the same or different, each represents a hydrogen or halogen atom, an amine or substituted amine group, or an unsubstituted or substituted straight or branched chain aliphatic group

or a salt thereof,

which comprises reacting a compound of the general formula II

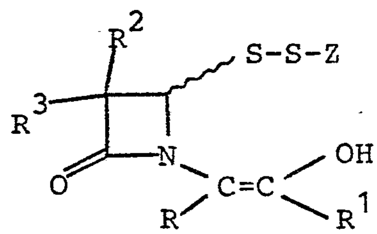

(II)

in which R, $R^1$, $R^2$ and $R^3$ are as defined above, and

Z represents an unsubstituted or substituted aromatic heterocyclic radical with up to 15, preferably up to 9, carbon atoms and at least one ring nitrogen atom and optionally a further ring heteroatom, which radical is bonded to the dithio group by one of its ring carbon atoms, which is bonded to a ring nitrogen atom by a double bond, or

Z represents an acyl radical derived from an organic carboxylic acid or thiocarboxylic acid,

with a tervalent organophosphorus compound and, preferably, simultaneously or subsequently with a thiophilic agent as

hereinafter defined, and, if desired, carrying out any one or more of the following steps in any appropriate order:

(i) converting an ester into another ester or the free acid,

(ii) converting a free acid into an ester or a salt,

(iii) converting a salt into the free acid or another salt.

A compound of formula I may have, independently, the R or S configuration at positions 5 and 6, i.e. a compound may be 5R, 6R; 5R, 6S; 5S, 6R; or 5S, 6S. Moreover, $R^2$ may be *trans* to the 1-oxa group with $R^3$ *cis* to the 1-oxa group, or *vice versa*. The present invention accordingly includes the production of any of the various possible isomers of the compound of formula I, in pure form or in admixture with any one or more other isomer(s). A resulting mixture of isomers may be resolved in the normal manner, if desired.

An aliphatic group $R^1$ is, for example, an alkyl, alkenyl or alkynyl group having up to 8 carbon atoms, and especially up to 4 carbon atoms.

A group $R^1$ may be substituted by one or more substituents, as appropriate. Examples of substituents are halogen atoms; oxo groups; hydroxyl and mercapto groups, alkoxy and alkylthio groups; alkylcarbonyl groups; carboxy, alkoxycarbonyl and alkylthiocarbonyl groups; alkanoyloxy and alkanoylthio groups; carbamoyl and carbamoyloxy groups and carbamoyl and carbamoyloxy groups substituted on the nitrogen atom by one or two groups selected from alkyl and aryl groups and the corresponding unsubstituted and substituted groups in which the oxygen atom or each or either oxygen atom is replaced by a sulphur atom; nitro, cyano and azido groups; amido and imido groups; imino, amino, mono- and di-alkylamino, mono- and di-arylamino groups, and N,N-alkylarylamino groups; acylamino groups; sulphinyl, sulphonyl and sulphonamido groups; cyclalkyl groups; aryl, aryloxy, arylthio, aryloxycarbonyl, arylthiocarbonyl, arylcarbonyloxyl, arylcarbonylthio, aralkoxycarbonyl, aralkylthiocarbonyl, aralkylcarbonyloxy, aralkylcarbonylthio, aralkoxy,

and aralkylthio groups; aromatic and non-aromatic heterocyclic groups, for example, having one or more heteroatoms, for example, up to 4 hetero atoms, which may be the same or different, selected from nitrogen, oxygen and sulphur atoms, and preferably up to 14 atoms in total, and the corresponding aromatic and non-aromatic heterocyclicoxy groups and aromatic and non-aromatic heterocyclicthio groups.

Examples of aromatic heterocyclic groups are 1-methyl-imidazol-2-yl, 1,3-thiazol-2-yl, 1,3,4-thiadiazol-2-yl, 1,3,4,5-thiatriazol-2-yl, 1,3-oxazol-2-yl, 1,3,4,5-oxatriazol-2-yl, 1,3,4,5-tetrazol-2-yl, 2-quinolyl, 1-methyl-benzimidazol-2-yl, benzoxazol-2-yl and benzthiazol-2-yl groups, and the corresponding 2-yl-oxy and 2-yl-thio groups.

Any substituent of $R^1$ that is itself capable of substitution may be substituted, for example, by any one or more of the substituents described above. Alkyl groups are preferably alkyl groups having up to 8 and especially up to 4 carbon atoms.

The term "lower" as used herein denotes a molecule, group or radical having up to 8 carbon atoms, and especially up to 4 carbon atoms. Unless stated otherwise, halogen atoms are fluorine, chlorine, bromine and iodine atoms. The term "known" means in actual use in the art or described in the literature of the art.

$R^1$ preferably represents a methyl group or a substituted methyl group, preferably substituted by one of the groups described above, for example, a methyl group having one or more substituents, for example, bromomethyl, a methoxy methyl group; an etherified or esterified methyl group, for example, a lower alkoxymethylene group, for example, a methoxymethylene group, or a lower alkanoyloxymethylene group, for example, a methylcarbonyloxymethylene group; a methyl group substituted by an azide, nitrile or thiocyanate group; a methyl group substituted by a -SHet group in which Het represents an aromatic or non-aromatic heterocyc-

lic group especially an aromatic heterocyclic group as mentioned above; or a methyl group substituted by an amino group or by a mono- or di-lower alkylamino, mono- or di-arylamino or lower alkyl-arylamino group; or a methyl group substituted by an acylamino group.

Preferably one of the radicals $R^2$ and $R^3$ represents a hydrogen or halogen atom with the other representing an unsubstituted or substituted, straight or branched chain aliphatic group. An aliphatic group may be a straight or branched chain lower alkyl, alkenyl or alkynyl group, for example, a methyl, ethyl or vinyl group. An aliphatic group $R^2$ or $R^3$ may be substituted as described above for $R^1$. Preferred substituents for an aliphatic group $R^2$ or $R^3$ are amino, mercapto and hydroxyl groups, which may themselves be substituted, for example, by one of the following groups: $-R^{10}$, $-CO-NR^{10}R^{11}$, $-CO-R^{10}$, $-CO-OR^{10}$, $-SO_2-R^{10}$, $-SO_2-OR^{10}$, $-SO_3^{\ominus}$, and, in the case of an amino group, $-R^{10}R^{11}$, in which groups $R^{10}$ and $R^{11}$, which may be the same or different, if appropriate, each represents an alkyl group, especially a lower alkyl group, an aryl group or an aralkyl group, especially an aryl-lower alkyl group. Furthermore, a nitrogen atom present as a direct substituent of $R^2$ or $R^3$ or present in the group $-CO-NR^{10}R^{11}$ may be part of an aromatic or non-aromatic heterocyclic ring.

$R^2$ or $R^3$ preferably represents a hydrogen atom and the other represents a lower alkyl group or a 1-hydroxyl-lower alkyl group. Especially preferred are those compounds in which $R^2$ or $R^3$ represents a hydrogen atom and the other represents a methyl, ethyl, hydroxy-methyl or 1-hydroxyethyl group, a chlorine, bromine or iodine atom, or an ethylidene or vinyl group.

A substituted amino group at position 6 may be a mono- or di-alkyl amino group, especially a mono- or di-lower alkylamino group, an acylamino group, or a nitrogen-containing heterocyclic group.

The stereochemistry at the 5-position is preferably the same as that found in naturally occuring penicillins,

and the 6-substituent is preferably trans to the 1-oxa group. An aliphatic group may be jointed to the 6-position of the oxapenem by a double bond in either the E or the Z configuration, for example, $R^2$ or $R^3$ may represent an E- or Z-alkylidene or alkenylidene group, for example, an E- or Z-ethylidene group.

An esterified carboxyl group R i.e. a group -COOR is, for example, an ester formed with an unsubstituted or substituted aliphatic, cycloaliphatic, cycloaliphatic-aliphatic, aryl, araliphatic, heterocyclic or heterocyclic-aliphatic alcohol having up to 20 carbon atoms or is, for example, a silyl or stannyl ester.

The ester moiety may be removable under acidic, neutral or basic conditions to give the free acid. In some cases it may be preferable if R represents an ester group that is removable under physiological conditions, that is to say, the ester group is split off in vivo to give the free acid, for example, an acyloxymethoxy ester, e.g. pivaloyloxy-methoxy ester, an aminoalkaneyloxy methoxy ester, for example, an L-glycyloxymethoxy, L-valyloxymethoxy or L-leucyloxy methoxy ester, or a phthalidyloxy ester, or an optionally substituted 2-aminoethoxy ester, for example, a 2-diethyl-amino-ethoxy or 2-(1-morpholino)-ethoxy ester. Further examples of esters are benzyl, p- and o-nitrobenzyl esters, benzhydryl, o-nitrobenzhydryl esters.

R' may represent, for example a straight or branched chain substituted or unsubstituted alkyl, alkenyl or alkynyl group having up to 18 carbon atoms, preferably up to 8 carbon atoms, and especially up to 4 carbon atoms, for example, a methyl, ethyl, n-propyl, iso-propyl, n-butyl, sec-butyl, iso-butyl, tert-butyl, n-pentyl, n-hexyl, allyl, or vinyl group.

An aliphatic group R', especially a methyl group, may be substituted by a cycloalkyl, aryl or heterocyclic group, or R may itself represent a cycloalkyl, aryl or heterocyclic group.

A cycloaliphatic group R' may have up to 18 carbon

atoms and is, for example, a cyclopentyl, cyclohexyl or adamantyl group. An aryl group may have up to 12 carbon atoms and may have two or more fused rings. An aryl group R' is, for example, an unsubstituted or substituted phenyl group, and an unsubstituted or substituted aralkyl group is, for example, a benzyl, p-nitrobenzyl or benzhydryl group.

A heterocyclic group may have one or more hetero atoms, selected from oxygen, nitrogen and sulphur, and up to 14 atoms in total. A heterocyclic group is, for example, an oxygen-containing heterocyclic group, for example, a tetrahydropyranyl or phthalidyl group.

A stannyl group R' may have up to 24 carbon atoms, for example, R' may represent a stannyl group having three substituents, which may be the same or different, selected from alkyl, alkenyl, cycloalkyl, aryl, aralkyl, alkoxy and aralkoxy groups, for example, alkyl groups having up to 4 carbon atoms, for example n-butyl groups, phenyl and benzyl groups, especially three n-butyl groups.

A silyl group R' may also have up to 24 carbon atoms and three substituents, which may be the same or different, selected from alkyl, alkenyl, cycloalkyl, aryl and aralkyl groups, for example, alkyl groups having up to 4 carbon atoms, for example, methyl and t-butyl groups.

Any group R' that is capable of substitution may be substituted. Examples of substituents are given above in relation to $R^1$. Substituents for phenyl groups are, for example, as described above in relation to preferred groups $R^1$.

The group R' may be removable by hydrolysis, by reduction or by enzyme action to give the free acid. A group R' that may be removed readily without substantial degradation of the rest of the molecule is particularly useful as a carboxyl protecting group. Examples of esters that are readily split by reduction are arylmethyl esters, for example, benzyl, p-nitrobenzyl, benzhydryl and trityl ester. Reduction of an ester, for example, an arylmethyl ester,

may be carried out using hydrogen and a metal catalyst for example, a noble metal, for example, platinum, pal: dium or rhodium, which catalyst may be supported, for example, on charcoal or kieselguhr.

A p-nitrobenzyl ester may be split, alternatively first by reduction of the nitro group, and then by hydı lysis. The nitro group may be reduced, for example, u: a metal reducin agent, for example, zinc in acetic aci( aqueous tetrahydrofuran or acetone. The pH should be maintained within the range of from 3 to 6, preferably from 4 to 5.5, preferably by the use of aqueous hydrocl ric acid. Other reducing agents are, for example, alur num amalgam in a moist ether, for example, tetrahydrofı an iron and ammonium chloride in an aqueous ether, for example, aqueous tetrahydrofuran. Reduction of the ni group is followed by hydrolysis which may occur in sitı during reduction of the nitro group or which may be carried out subsequently by treatment with an acid.

A stannyl ester, for example, a tri-n-butyl stann: ester, may be split readily by hydrolysis, for example solvolysis, for example, using water, an alcohol, a phe or a carboxylic acid, for example, acetic acid.

In some cases it may be preferable if the ester gı is removable under physiological conditions, that is t( say, the ester group is split off in vivo to give the : acid, for example, an acyloxymethyl ester, e.g. an acet methyl or pivaloyloxymethyl ester, an aminoalkanyloxym( ester, for example, an L-glycyloxymethyl, L-valyloxymet or L-leucyloxymethyl ester, or a phthalidyl ester, or a optionally substituted 2-aminoethyl ester, for example, 2-diethylamino-ethyl or 2-(1-morpholino)-ethyl ester.

Preferred esters are the p-nitrobenzyl, phthalidy: and pivaloyloxymethyl esters.

An ester of formula I, or of any other free acid ( cribed herein, may be prepared by reaction with an alc( phenol or stannanol or a reactive derivative thereof. reaction is preferably carried out under mild conditio

BAD ORIGINAL

in order to prevent rupture of the ring or ring system, for example, under neutral or mild acidic or basic conditions, and at temperatures within the range of from -70° to +35°C.

An alkyl, alkoxyalkyl or aralkyl ester may be prepared by reaction of an acid of formula I or any other free acid with the appropriate diazoalkane or diazoaralkane for example, diazomethane or diphenyldiazomethane. The reaction is preferably carried out in an ether, ester or halogenhydrocarbon as solvent, for example, in diethyl ether, ethyl acetate or dichloromethane. In general, temperatures below room temperature and preferred, for example, from -15° to +15°C.

An ester derived from an alcohol may also be produced by reaction of a reactive derivative of the alcohol, for example, a halide, for example a chloride, bromide or iodide, or a hydrocarbonsulphonyl derivative, for example, a mesyl or tosyl ester, with a salt of an acid of formula I or another free acid described herein for example, an alkali or alkaline earth metal salt, for example, a lithium, sodium, potassium, calcium or barium salt or an amine salt, for example, a triethylammonium salt. This reaction is preferably carried out in a substituted sulphoxide or amide solvent for example, in dimethyl sulphoxide, dimethylformamide or hexamethylphosphoramide or, alternatively, an ester may be prepared by reaction of the acid with the alcohol in the presence of a condensing agent, for example, dicyclohexylcarbodiimide.

A stannyl ester may be formed by reaction of a carboxylic acid of formula I or another free acid described herein, or a salt thereof with a reactive tetravalent tin compound, especially a trialkyl tin oxide.

An aromatic heterocyclic radical Z may be monocyclic or bicyclic and may be substituted, for example, by a lower alkyl group, for example, a methyl or ethyl group, a lower alkoxy group, for example, a methoxy or ethoxy group, a halogen atom, for example, a fluorine or chlorine atom, or

an aryl group, for example, a phenyl group.

A heterocyclic radical Z is, for example, a monocyclic five-membered thiadiazacyclic, thiatriazacyclic, oxadiazacyclic or oxatriazacyclic radical of aromatic character, especially a monocyclic five-membered diazacyclic, oxacyclic or thiazacyclic radical of aromatic character, and above all, the corresponding benzdiazacyclic, benzoxazacyclic or benzthiazacyclic radicals, wherein the heterocyclic part is five-membered and is of aromatic character.

In radicals Z a substituable ring nitrogen atom can be substituted, for example, by a lower alkyl group. Examples of such groups Z, are 1-methyl-imidazol-2-yl, 1,3-thiazol-2-yl, 1,3,4-thiadiazol-2-yl, 1,3,4,5-thiatriazol-2-yl, 1,3-oxazol-2-yl, 1,3,4-oxadiazol-2-yl, 1,3,4,5-oxatriazol-2-yl, 2-quinolyl, 1-methyl-benzimidazol-2-yl, benzoxazol-2-yl and especially benzthiazol-2-yl groups. Z may also prepresent an acyl radical or organic carboxylic or thiocarboxylic acid, for example, an unsubstituted or substituted aliphatic, cycloaliphatic, araliphatic or aromatic acyl or thioacyl group having up to 18, preferably up to 10, carbon atoms, for example, lower alkanoyl groups, for example acetyl and propionyl groups, lower thioalkanoyl groups, for example thioacetyl and thiopropionyl groups, cycloalkanecarbonyl groups, for example a cyclohexane-carbonyl group, cycloalkanethiocarbonyl group, for example, a cyclohexanethiocarbonyl group, benzoyl, thiobenzoyl, naphthylcarbonyl, and naphthylthiocarbonyl groups, heterocyclic carbonyl and thiocarbonyl groups, for example, 2-, 3- or 4-pyridylcarbonyl, 2- or 3-thenoyl, 2- or 3-furoyl, 2-, 3-, or 4-pyridyl-thiocarbonyl, 2- or 3-thiothenoyl, and 2- or 3-thiofuroyl groups, and corresponding substituted acyl and thioacyl groups, for example acyl and thioacyl groups monosubstituted or polysubstituted by lower alkyl groups, for example, methyl groups, halogen atoms, for example, fluorine and chlorine atoms, lower alkoxy groups, for example, methoxy groups, aryl groups for example phenyl groups, and aryloxy groups, for example phenyloxy groups.

Z preferably represents a

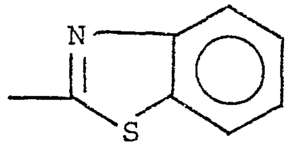 or 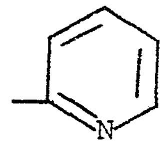 group.

The tervalent organophosphorus compound is especially one of the general formula

$$PR^4R^5R^6$$

wherein $R^4$, $R^5$ and $R^6$, which may be the same or different, each represents an unsubstituted or substituted hydrocarbon group, for example, a straight or branched chain aliphatic for example, alkyl group, an unsubstituted or substituted cycloaliphatic for example cyclopentyl or cyclohexyl group, an unsubstituted or substituted aryl for example, phenyl group; or an unsubstituted or substituted hydrocarbon group in which one or more carbon atoms are replaced by hetero atoms, especially nitrogen, oxygen and sulphur atoms, for example, alkoxy groups, amine groups, and aromatic and non-aromatic heterocyclic groups. Preferred tervalent organophosphorus compounds are triphenylphosphine, tributylphosphine, trimethylphosphite and triethylphosphite.

A further preferred group of tervalent organophosphorus compounds are those in which, in $PR^4R^5R^6$ one or more of the groups $R^4$, $R^5$ and $R^6$ comprises an insoluble polymer, which aids removal after the reaction. Generally one polymeric substituents is adequate.

Another preferred group of tervalent organophosphorus compounds are those in which, in $PR^4R^5R^6$ one or more of the groups $R^4$, $R^5$ and $R^6$ comprise a cationic or anionic centre, for example, a quaternary ammonium group or a carboxylate or sulphate group. The presence of a charged group assists removal of the resulting organophosphorus sulphide, for example, by partition or by absorption on an insoluble ion exchange resin or by extaction into an aqueous solution at an appropriate pH, when the sulphide is water soluble.

The thiophilic agent is especially a thiophilic metal salt, for example, a salt of an element of Group Ib, IIb or VIII of the Periodic Table of the Elements (cf. Advanced Inorganic Chemistry, F.A. Colter and G. Wilkinson, Interscience), for example, a silver, copper, zinc, nickel, iron, cadmium, mercury or cobalt thiophilic salt, silver and copper salts being preferred. Examples of preferred thiophilic salts are (i) $AgNO_3$, $AgOSO_2CF_3$, $AgOSO_2Me$, $AgBF_4^{\ominus}$, $AgFF_6^{\ominus}$, CuCl, CuBr, $CuCl_2$, $CuBr_2$, $CuOSO_2Me$, $CuOSO_2CF_3$, $CuSO_4$, $Cu(NO_3)_2$ and the corresponding nickel, zinc, iron, and cobalt salts; and (ii) $Ag_2CO_3$, AgO, $Cu(acac)_2$, $Cu(CO_3)_2$, $Cu_2CO_3$, $CuOR^7$ in which $R^7$ represents an alkyl group or cycloalkyl group for example, comprising up to 8 carbon atoms, preferably $CH_3$, $C_2H_5$, t-butyl. Silver acetate is particularly preferred.

As a proton is liberated in the course of the reaction with the thiophilic agent, it is desirable that this is removed by a base so the reaction can go to completion. The base preferably has a $pK \leq 4$, so if the metal salt is itself sufficiently basic, for example, the salts of group (ii) above, it is not necessary to incorporate a further base in the reaction mixture. If, however, the metal salt is only weakly basic, for example, the salts of group (i) above, then it is preferable to use a further base, having a pK 4. The term "thiophilic agent" is used herein to mean a thiophilic metal salt alone, or a thiophilic metal salt plus base, as appropriate.

The base may be inorganic or organic, and preferred organic bases are pyridine, lower alkyl substituted pyridines, alkylamine substituted pyridines, piperidine and lower alkyl substituted piperidine, e.g. 2,2,6,6-tetramethylpiperidine, and trisubstituted amines, for example, trialkylamines, for example triethylamine and ethyldiisopropylamine, and N-alkyl-arylamines, for example, 1,8-bis-(dimethylamine) naphthalene, or N,N-dimethylphenylamine. Examples of preferred inorganic bases are metal hydrides, e.g. sodium hydride, and metal carbonates, for example, sodium carbonate.

According to the preferred method of carrying out the process, the starting material is reacted with the tervalent organophosphorus compound and simultaneously, a thiophilic agent, especially a metal salt, and where appropriate, a base.

The process of the invention is preferably carried out in the presence of an appropriate, inert, anhydrous solvent or diluent. Any solvent or diluent can be used which is inert to the sulphide, the tervalent phosphorus compound and the final product. Examples of solvents and diluents are benzene, toluene, acetonitrile, ethyl acetate, dichloromethane, chloroform, and dioxane. Mixtures of two or more solvents and diluents may also be employed. The process may be carried out at a temperature of from $-40^{\circ}$ to $+80^{\circ}$, preferably from $0^{\circ}$ to $+20^{\circ}$.

It is preferable to use 1 equivalent of the tervalent organophosphorus compound, and to incorporate this compound and the thiophilic agent in the reaction mixture.

The treatment of the disulphide of formula II with an organophosphorus compound and, simultaneously or subsequently, a thiophilic agent, gives the corresponding compound of formula I as the major reaction product. Treatment with an organophosphorus compound alone, however, generally gives a mixture of the corresponding compound of formula I and the monosulphide corresponding to formula II, i.e. a compound of the general formula IIa

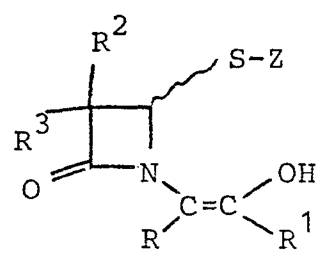

(IIa)

in which R, $R^1$, $R^2$, $R^3$ and Z are as defined above. This mixture may be treated with a thiophilic agent, or the compound of formula I and the monosulphide of formula IIa

may be separated and the latter treated with a thiophilic agent to produce a compound of formula I.

The invention accordingly also provides a process for the production of a compound of the general formula I as defined above or a salt thereof, which comprises treating a compound of the general formula IIa

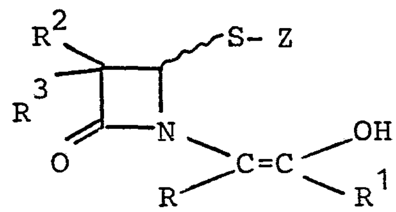

(IIa)

in which R, $R^1$, $R^2$, $R^3$ and Z are defined as above, with a thiophilic agent, as hereinbefore defined and, if desired, carrying out any one or more of the further steps (i) to (iii). The various radicals R to $R^3$ and Z preferably have the preferred meanings given above, and the preferred thiophilic agents and reaction conditions are as indicated above.

A compound of formula II may be produced by converting a compound of formula III

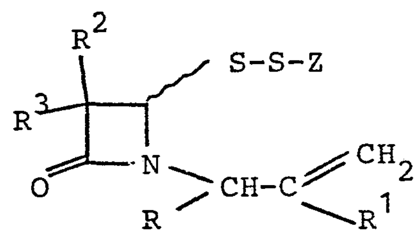

(III)

in which R, $R^1$, $R^2$, $R^3$ and Z are as defined above, into the corresponding enol.

This is preferably carried out by ozonolysis, generally via intermediate ozonide of the general formula

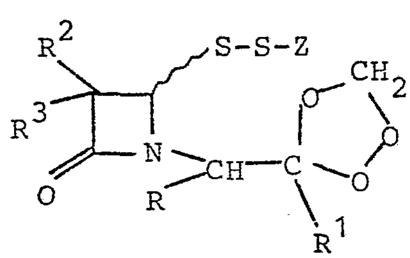

(IIb)

Ozone is usually employed in the presence of a solvent, for example, an alcohol, for example a lower alkanol, for example methanol or ethanol, a ketone, for example a lower alkanone, for example acetone, an optionally halogenated aliphatic, cycloaliphatic or aromatic hydrocarbon, for example a halogeno-lower alkane, for example methylene chloride or carbon tetrachloride, an ester, for example methyl acetate or ethyl acetate, or a mixture of two or more solvents, including an aqueous mixture, and with cooling or slight warming, for example at temperatures of from $-90^{\circ}C$ to $+40^{\circ}C$, preferably from $-60^{\circ}C$ to $+0^{\circ}$.

An ozonide intermediate IIb can be split by reduction to give a compound of the formula III for which it is possible to use catalytically activated hydrogen, for example hydrogen in the presence of a heavy metal hydrogenation, catalyst, for example a nickel catalyst, or a palladium catalyst, preferably on a suitable carrier, for example, calcium carbonate or charcoal, or a chemical reducing agent, for example, a reducing heavy metal, including a heavy metal alloy or amalgam, for example zinc, in the presence of a hydrogen donor, for example, an acid, for example acetic acid, or an alcohol, for example a lower alkanol, a reducing inorganic salt, for example an alkali metal iodide, for example sodium iodide, in the presence of a hydrogen donor, for example, an acid, for example acetic acid, a reducing sulphide compound for example, sulphur dioxide or a di-lower alkyl sulphide, for example dimethyl sulphide, a reducing organic phosphorus compound, for example a phosphine, which can optionally contain substituted aliphatic or aromatic hydrocarbon radicals as substituents, for example tri-lower alkylphosphines, for example tri-n-butylphosphine, or triarylphosphines, for example triphenylphosphine, also phosphites which contain optionally substituted aliphatic hydrocarbon radicals as substituents, for example tri-lower alkyl phosphites, usually in the form of the corresponding alcohol adduct compounds, for example trimethyl phosphite, or phosphorus acid triamides which

contain optionally substituted aliphatic hydrocarbon radicals as substituents, for example hexa-lower alkyl phosphorus acid triamides, for example hexamethyl-phosphorus acid triamide, the latter preferably in the form of a methanol adduct, or tetracyanoethylene. The splitting of the ozonide, which is usually not isolated, is normally carried out under the conditions which are employed for its manufacture, that is to say in the presence of a suitable solvent or solvent mixture, and with cooling or slight warming.

The ozonolysis is preferably carried out in ethyl acetate or dichloromethane which comprises from 0 to 50 % by volume of methanol. A mixture of 75 % dichloromethane and 25 % methanol is particularly preferred. The preferred reducing agents are dimethyl sulphide and sulphur dioxide.

The ozonolysis may result, *inter alia*, in a mixture of isomers IIc and IId about the double bond, i.e.

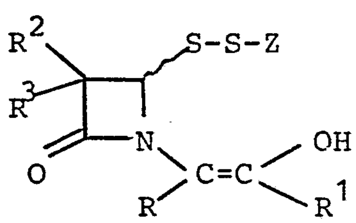

(IIc)

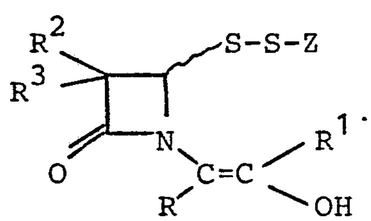

(IId)

These isomers are readily interconvertible, and compounds of the formulae IIc and IId can also exist in the tautomeric keto form.

It is usual for the subsequent reaction(s) to be carried out on the mixture of isomers, and keto tautomer if present. The presence of the keto tautomer does not adversely affect subsequent reactions.

A compound of formula IIa may be produced, as indicated above, by desulphurizing a compound of formula II using a tervalent organophosphorus compound, or it may be produced by converting a compound of formula IIIa

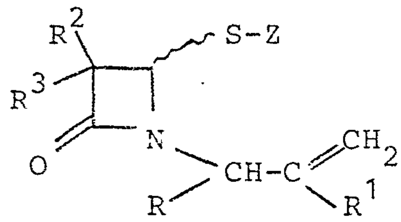

(IIIa)

in which R, $R^1$, $R^2$, $R^3$ and Z are as defined above, into the corresponding enol, preferably by ozonolysis as described above. The compound of formula IIIa may be produced by desulphurizing the corresponding compound of formula III using a tervalent organophosphorus compound as previously described. The reaction is generally conducted in the presence of an inert solvent or diluent, for example, a saturated hydrocarbon, e.g. pentane or hexane; a saturated cyclohydrocarbon, e.g. cyclohexane; an aromatic hydrocarbon, e.g. benzene, or toluene; acetonitrile or ethylacetate; a chlorinated hydrocarbon, e.g. dichloromethane, or chloroform; or an oxygenated hydrocarbon, e.g. ethylacetate, acetone, ethanol, tetrahydrofuran or dioxane. Mixtures of two or more solvents may be employed. Preferred solvents are methylene chloride and chloroform, and the preferred temperature is from -40 to +80°C, especially from -20 to +20°C.

Various possible routes for producing a compound of formula I are illustrated by way of example in the following reaction scheme.

BAD ORIGINAL

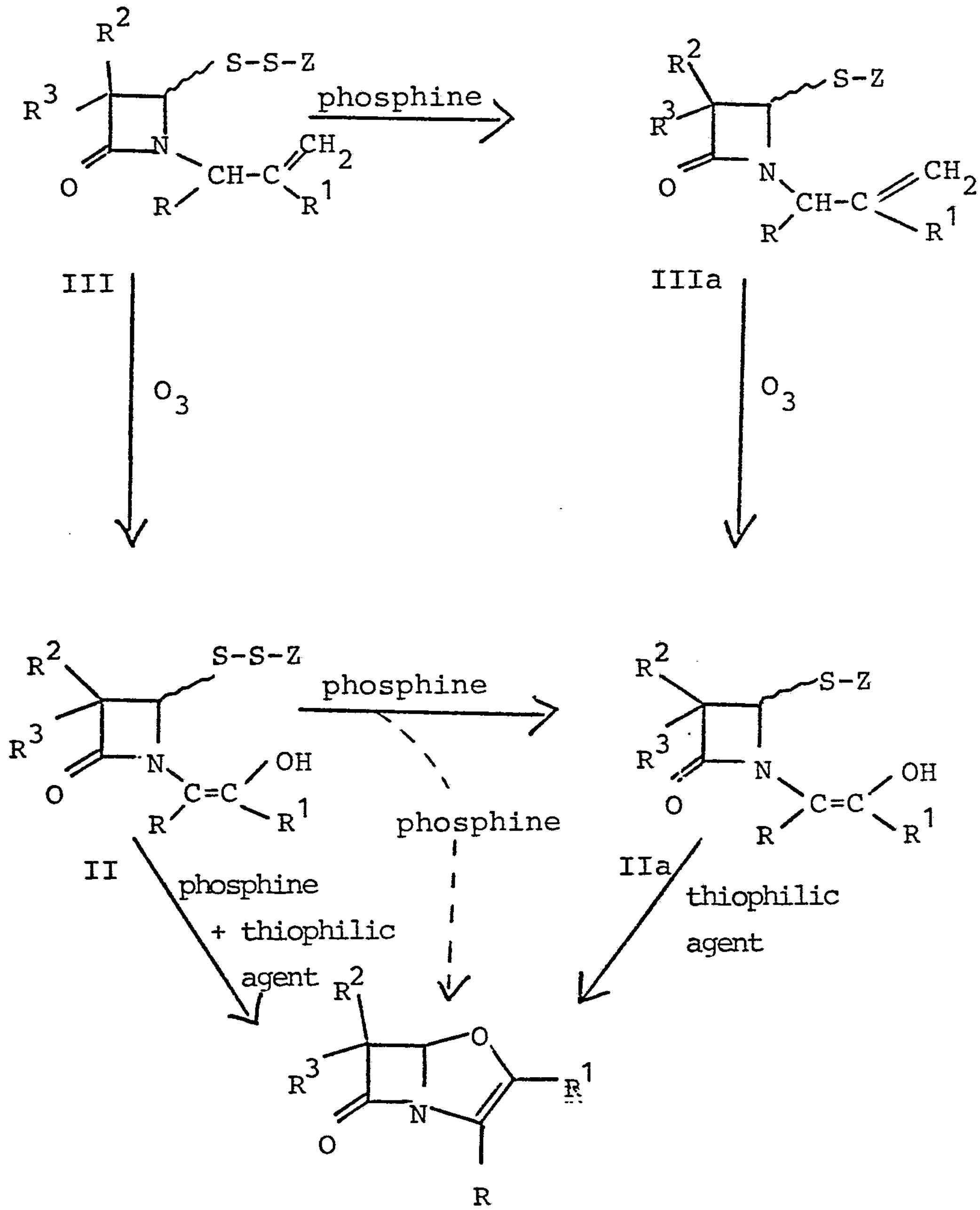


III IIIa II IIa

I

A compound of formula III in which $R^1$ represents a methyl group is preferably produced by reacting a compound of formula IVa

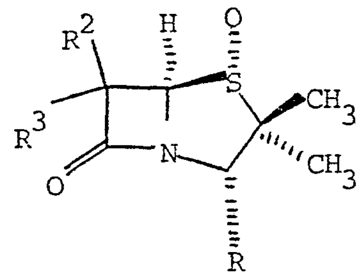
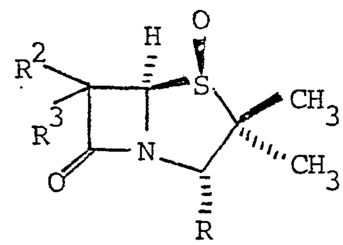

(IVa)

in which R, $R^2$ and $R^3$ are as defined above with a compound of formula V

H - S - Z (V)

in which Z is as defined above.

This reaction may be carried out by merely heating the compound of formula IVa with the compound of formula V, preferably in a nitrogen or argon atmosphere, in an inert solvent or diluent at a temperature from 50 to 150°, especially from 80 to 120°. Suitable solvents are those which posses a sufficiently elevated boiling point to achieve the necessary reaction temperature and in which the starting materials and product are stable at the temperature of the reaction. Examples of solvents are benzene, toluene, ethylacetate, acetonitrile, dioxane, N,N-dimethyl formamide and N,N-dimethylacetamide.

When a compound of formula III where R is other than a methyl group is required this is preferably produced by reacting a compound of the formula IVb

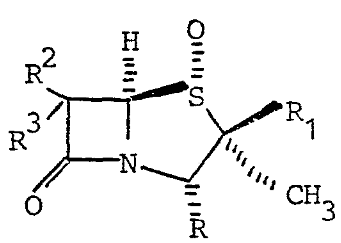
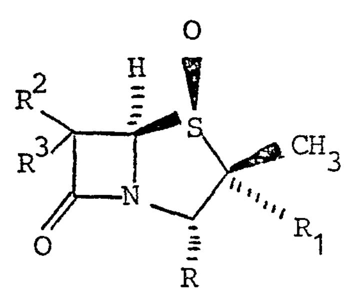

(IVb)

in which R, $R^1$, $R^2$ and $R^3$ are as defined above and $R^1$ preferably represents a substituted methyl group, with a com-

BAD ORIGINAL

pound of formula H-S-Z in which Z is as defined above.

A substituted methyl group is, for example, a $-CH_2OH$ group, a $-CH_2SR^{12}$ group in which $R^{12}$ represents an aromatic or non-aromatic heterocyclic group, especially one of those mentioned above as a substituent for $R^1$, a $-CH_2$ alkyl or $-CH_2$ aryl group, a $-CH_2Hal$ group in which Hal represents a halogen atom, a $-CH_2NHCOR^{13}$ group in which $R^{13}$ represents a lower alkyl group which may be substituted as defined above for $R^1$, a $-CH_2N_3$, $-CH_2SCN$ or $-CH_2CN$ group, or a $-CH_2OCOR^{13}$ group in which $R^{13}$ is as defined above, with a compound of formula V, preferably under the conditions described above.

Some Examples of analogues of compounds possessing the above substituted penam ring system are described in U.S. patents nos. 4 081 443 and 4 056 521 and compounds of formula IVb may be produced analogously to the process described therein.

The compound of formula IVa is preferably produced from a compound of formula IV

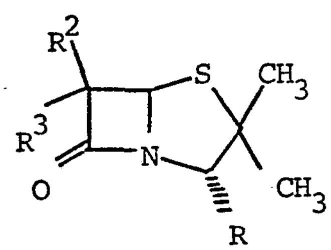

(VI)

in which R, $R^2$ and $R^3$ are defined as above, by means of oxidation.

The oxidation may be carried out by any method suitable for oxidising sulphides to the corresponding sulphoxides. Oxidising agents are, for example, hydrogen peroxide, periodates, e.g. sodium periodate, ozone, peracids, e.g. peracetic acid, perbenzoic acid, substituted perbenzoic acids, e.g. n-chloroperbenzoic acid and permanganate salts, e.g. potassium permanganate. Preferred oxidising agents are metachloroperbenzoic acid and hydrogen peroxide.

The oxidation is preferably conducted in an inert solvent at a preferred temperature of from -20° to +30°.

Preferred solvents are ethyl acetate, methylene chloride, chloroform, acetonitrile, and lower alcohols, for example methanol and ethanol.

A compound of formula VI may be prepared in several ways, for example, a compound in which $R^2$ and $R^3$ both represent hydrogen atoms may be prepared by catalytically hydrogenating a compound of the general formula VII

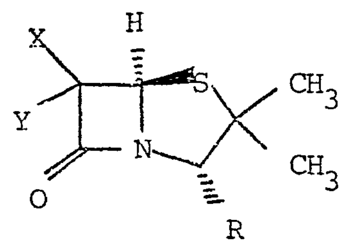

(VII)

in which X and Y are the same and each represents a halogen atom, or X represents a hydrogen atom and Y represents a halogen atom, and R is as defined above.

An example of such a process in which R represents a free carboxyl group is given in J.Chem.Soc. 2623 (1969).

A compound of formula VI in which $R^2$ or $R^3$ represents a hydrogen atom and the other represents an organic substituent as defined above, or both $R^2$ and $R^3$ represent organic substituents, may be prepared by introducing the appropriate group(s) into a compound of formula VII. An example of such process is given in J.Org.Chem. 42, 2960 (1977) where a hydroxyethyl group is introduced into a compound of the formula VII wherein X and Y each represents a bromine atom or X represents a hydrogen atom and Y an iodine atom by the use of an organometallic reagent, for example, *n*-butyllithium or methylmagnesium bromide in, for example, tetrahydrofuran or diethyl ether, to prepare a metalated compound of formula VIII, where X represents a hydrogen or bromine atom and the metal is $Li^+$, $MgBr^+$.

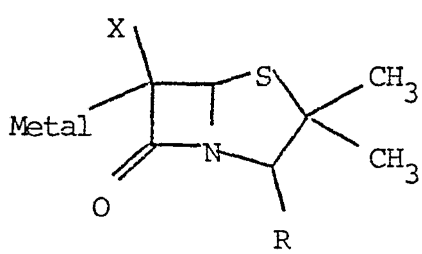

(VIII)

BAD ORIGINAL

Subsequent treatment of the metalated compound of formula VIII with acetaldehyde gives a compound of formula VI where R is as defined above, $R_2$ represents an 1-hydroxyethyl group and $R_3$ represents a bromine or hydrogen atom. This reaction may be carried out analogously using other aldehydes.

A compound of formula VI where $R_2$ represents an 1-hydroxyethyl group and $R_3$ a hydrogen atom can be further transformed, according to a procedure described in J.Amer. Chem.Soc. 3251, 100, (1978), to a compound of formula VI where $R_2$ represents an ethyl group and $R_3$ represents a hydrogen atom, i.e. the hydroxyl group of the hydroxyethyl group has been replaced by hydrogen.

Other methods of introducing, into a ß-lactam, alkyl groups, aralkyl groups and 1-hydroxyalkyl groups have been described in the chemical literature, for example Can.J. Chem. 3196, 50 (1972), Can.J.Chem. 3206 52 (1974) and Synthesis 746 (1978).

A compound of formula VI in which $R^2$ represents an unsubstituted or substituted alkyl or aralkyl group and $R^3$ represents a hydrogen atom or *vice versa* may generally be prepared as follows:

A compound of formula VII in which R preferably represents an esterified carboxyl group is treated with an organo-metallic reagent of the formula $M^{\oplus}R_a^{\ominus}$ in which $R_a^{\ominus}$ represents a lower alkyl or aryl anion, for example, a phenyl anion and $M^{\oplus}$ represents metal cation e.g. a lithium cation, a cuprous cation, a magnesium bromide, chloride or iodide cation, a zinc bromide, chloride or iodide cation, or a $ZnR^{14}$ cation, where $R^{14}$ represents a lower alkyl group, especially a methyl or ethyl group. Examples of such organometallic reagents are methyllithium, *n*-butyllithium, *sec*-butyllithium, *tert*-butyllithium, methylmagnesium bromide, *tert*-butylmagnesium bromide, *iso*propylmagnesium bromide, and diethyl zinc.

It is preferable that this treatment is conducted in a suitable, inert solvent or diluent, for example, a saturated hydrocarbon ether, for example, diethylether, or 1,2-dimethoxyethane; a saturated cyclic ether, for example tetrahydrofuran or 1,4-dioxane; or an aromatic hydrocarbon, e.g. toluene. The reaction may be conducted at temperatures from -120° to 0°, but the preferred temperature is from -80° to -60°. The resulting metalated compound of formula VIII where Metal has one of the meanings given above for $M^{\oplus}$, R is as previously defined and X represents a hydrogen or bromine atom is not usually isolated but is preferably treated in situ with a compound of the formula $R^2_a R^3_a C=O$, in which $R^2_a$ and $R^3_a$ have the meanings given for $R^2$ and $R^3$ and may additionally represent aryl. A metalatd compound of formula VIII wherein Metal is as defined above for $M^{\oplus}$ obtained with the use of any other suitable organometallic reagent may also be treated with a ketone $R^2_a R^3_a C=O$.

The resulting compound of formula IXa

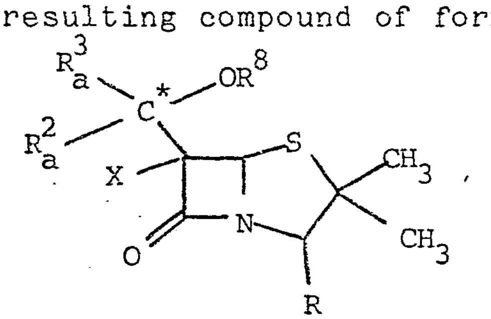

(IXa)

wherein $R^8$ has one of the meanings given above for $M^{\oplus}$ may be treated, for example in situ, with an acid for example acetic acid to give the corresponding compound of formula IXa where $R^8$ represents hydrogen atom.

A compound of formula IXa wherein X and $R^8$ each represents a hydrogen atom can be further transformed into a compound of formula IXb where the hydroxyl group has been replaced by a hydrogen atom.

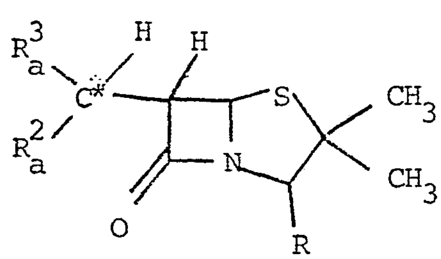

(IXb)

This process may be effected by treatment of a compound of general formula IXa wherein $R^8$ represents a hydro-

BAD ORIGINAL

gen atom with a compound which will activate the hydroxyl group. A preferred method is to prepare an alkyl sulphonyl derivative IXa wherein $R^8$ represents $SO_2R^9$, and $R^9$ represents an aromatic hydrocarbon radical, e.g. a phenyl, or 4-methylphenyl radical or a lower alkyl group, e.g. a methyl group, for example from a compound IXa wherein $R^8$ represents a hydrogen atom by treatment with an activated sulphonyl derivative $R^9SO_2X$ where X represents a halogen atom or $-OSO_2R^9$, i.e. a sulphonyl anhydride, in the presence of a base. An organic or inorganic base may be used, preferred organic bases being pyridine, alkylsubstituted pyridines, e.g. 2,6-dimethylpyridine, alylamino pyridine, e.g. 4-N,N-dimethylaminopyridine, trisubstituted amine, e.g. triethylamine, ethyldi-iso-propylamine, and alkyl, aryl amines, e.g. N,N-dimethylaniline. Preferred inorganic bases are metal hydrides, e.g. sodium hydride, and metal carbonates, e.g. sodium carbonate.

The reaction is optimally performed in a suitable inert solvent or diluent at temperatures from -40° to +40°C, preferably at temperatures of from -10° to +10°C. Examples of solvents and diluents are ethylacetate, benzene, toluene, acetonitrile, chloroform, and methylene chloride, dioxan and tetrahydrofuran.

A compound of formula IXa wherein $R^8$ represents $SO_2R^9$, $R^9$ being as defined above, may be further treated with an organic base, e.g. triethylamine, 1,4-diazobicyclo/2,2,2/-octane, to produce the chelactams of formula IXc

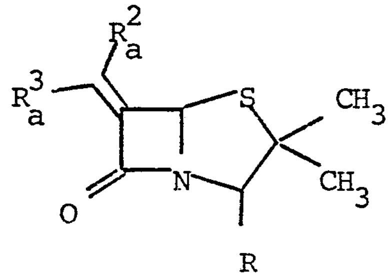

(IXc)

which may have the E- or Z-configuration or position 6.

The process may be conducted in an inert solvent or diluent, for example, benzene, toluene, ethyl acetate, or acetonitrile, and at a temperature of from 20° to 120°C, preferably at a temperature of about 80°C.

A compound of formula IXb may be prepared from a compound of formula IXc by means of reduction. The preferred. method of reduction is by catalytically activated hydrogen for example with Pd/C, Pd/$CaCO_3$, or $PtO_2$ in the presence of an inert solvent or diluent, for example, ethylacetate at temperatures of from 0 to 40°C and at a pressure of hydrogen of from 1 to 4 atmospheres. A Parr hydrogenator is preferably used for this reduction.

Compounds resulting from the above preferred procedures, i.e. compounds of formula IXa and IXb exist as 6R and 6S isomers, and compounds IXa and IXb also exist as R and S isomers at the starred carbon atom, where $R_a^2$ and $R_a^3$ are not the same.

The halogen atom present in the compounds IXa and IXd may be removed by reduction, for example, hydrogen in the presence of a noble metal catalyst, for example Pd/C, Pd/$CaCO_3$, Pt; a heavy metal alloy or amalgam for example, Zn/Ag; an organo-stannyl hydride, e.g. tri-n-butyl stannyl hydride. The reduction may be carried out under the conditions normally employed for the use of these reducing agents. The resulting compounds fall within the general formula VI.

The above methods relate to the interconversion of compounds of formulae VI and VII having different substituents $R^2$ and/or $R^3$. Such interconversion may be carried out in compounds of formula IV i.e. in the corresponding sulphoxides. Any method may be used provided that the sulphoxide group is not affected during the reaction, for example, there may be used any of the above methods that fulfil this criterion. Particularly preferred are those reactions involving reaction of a sulphoxide of formula IV with an organometallic reagent to give a metallated sulphoxide which is then treated with an aldehyde, for example, acetaldehyde or benzaldehyde, or with a ketone, for example, acetone. Such reactions may be carried out analogously to those described above in relation to compounds of formula VI. The starting material of formula IV is preferably the 6-monoiodo compound i.e. one of $R^2$ and R3 represents a hydrogen

atom and the other represents an iodine atom. The starting material of formula IV is preferably in the form of the p-nitrobenzyl or pivaloyloxymethyl ester.

Some compounds of formula VII are known and can be prepared according to known procedures. One such procedure which is described in J.Chem.Soc., 2623 (1969), involves the treatment of 6-amino penicillanic acid simultaneously with nitrous acid and a halogenating agent. The use of sodium bromide as the halogenating agent results in a compound of formula VII in which X represents a hydrogen atom, Y represents a bromine atom and R represents a free carboxyl group, with sodium iodide giving the corresponding compound in which Y represents an iodine atom, and bromine giving the dibromo analogue.

A compound of formula VII in which R represents $-CO_2CH_2Ph$, X represents a hydrogen atom and Y represents an iodine atom, and the dibromo analogous thereof may both be prepared by the process described in J.Org.Chem., 43, 2960 (1977).

Other compounds of formula VII may be prepared analogously.

6-Aminopenicillanic is an advantageous starting material as it is readily available and relatively inexpensive. It will be appreciated that the use of a compound analogous to 6-aminopenicillanic acid but having a different stereochemical configuration will result in the production of a compound of formula VII having the corresponding stereochemical configuration.

The analogues of the various intermediates of formulae VI to IX in which $R^1$ represents other than a methyl group may be prepared analogously to the above described intermediates, for example, starting from the appropriate analogue of 6-aminopenicillanic acid, or compound IVb may be produced as described in the above mentioned U.S. patents.

It is advisable to esterify a free carboxyl group in a compound of formula II or IIa prior to cyclisation as a free carboxyl group is nucleophilic and it is undesirable to have this nucleophilic group present in addition to the

hydroxyl group. Although an ester group may be introduced immediately prior to cyclisation, it is preferable to esterify the carboxyl group at an earlier stage in the preferred reaction sequence, for example, to esterify a free carboxyl group in a compound of formula VI or VII, to ensure that the carboxyl group does not take part in any of the subsequent reactions.

At each stage of the preferred reaction sequence, the desired compound may be isolated from the reaction mixture and, if desired, purified by appropriate techniques, for example, chromatography.

As indicated above, various intermediates may be produced in the form of mixtures of isomers of various kinds. Such a mixture may be resolved at any stage, or the isomeric mixture may be used *per se* for subsequent reactions. The isomers of formulae II and IIa that appear to cyclise are

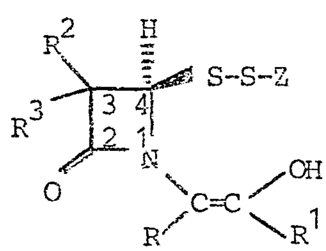

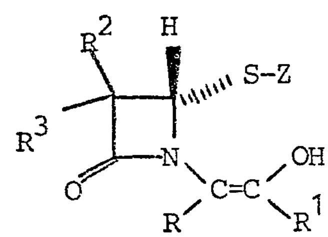

When $R^2$ and $R^3$ are other than hydrogen atoms, either may be *cis* or *trans* to the -SZ or -S-S-Z group, as mentioned above for formula I. A resulting mixture of isomers of formula I may be resolved if desired into the various isomers, by the usual methods.

The present invention provides a compound of the general formula X

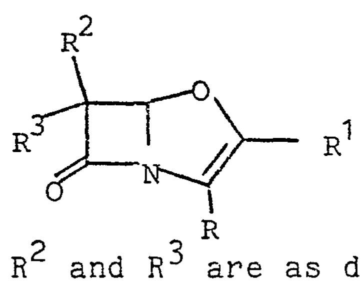

(X)

in which R, $R^1$, $R^2$ and $R^3$ are as defined for the general formula I, with the exception of those compounds in which $R^2$ or $R^3$ represents an amine or substituted amino group, directly bonded to the ring carbon atom and those in which

$R^2$ and $R^3$ both represent hydrogen atoms. Particularly preferred are those compounds in which $R^1$ represents a methyl group.

The invention further provides a compound of the general formula XI

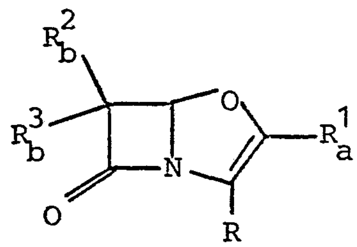

(XI)

in which $R_b^2$ and $R_b^3$ each represents a hydrogen atom, R is as defined for formula I and $R_a^1$ has the same meaning as $R^1$ in formula I, with the exception of vinyl groups, unsubstituted alkyl groups having up to 3 carbon atoms, methyl groups having one or two phenyl substituents, and ethyl groups substituted by a hydroxyl, ether, or acyl group, or an S-containing nucleophilic group.

The invention further provides a compound of the general formula XII

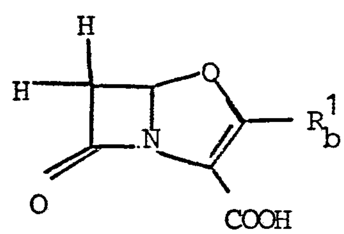

(XII)

in which $R_b^1$ represents an unsubstituted or substituted alkyl group having 3 or more carbon atoms, or a substituted methyl or substituted ethyl group.

The invention further provides a compound of the formula XIII

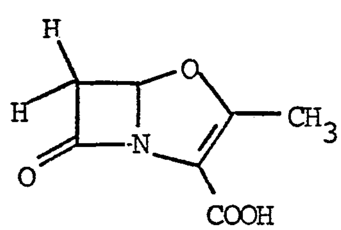

(XIII)

The invention further provides a compound of the generoal formula XIV

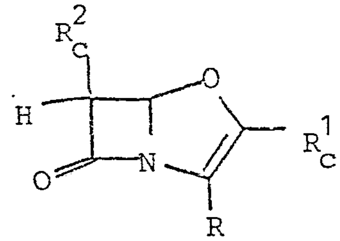

(XIV)

in which $R_c^2$ has the R or S configuration and represents a methyl, ethyl, hydroxymethyl or 1-hydroxyethyl group or a halogen atom, a hydroxymethyl or 1-hydroxyethyl group or a halogen atom preferably being trans to the 1-oxa group, and $R_c^1$ represents a methyl or substituted methyl group.

A substituted methyl group $R_b^1$ or $R_c^1$ is especially a $-CH_2N_3$ or $-CH_2SCN$ group, a $-CH_2NHCOR^{14}$ or $-CH_2OCOR^{14}$ group wherein $R^{14}$ represents a lower alkyl group which may be substituted as defined above for $R^1$, a $-CH_2Hal$ or $-CH_2SHet$ group wherein Hal represents a halogen atom and Het represents an aromatic or nonaromatic heterocyclic group which may be substituted, especially one of those described above as substituents for $R^1$.

The following are examples of compounds of the invention:

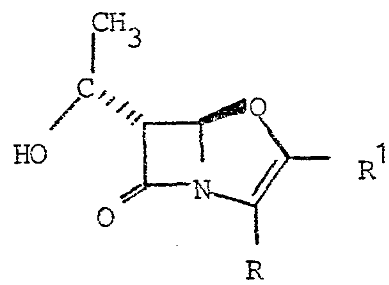

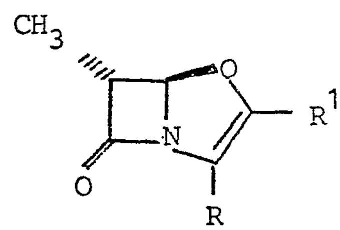

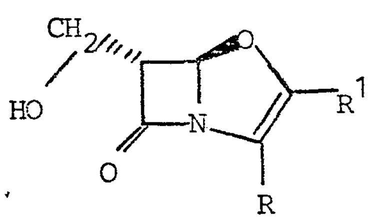

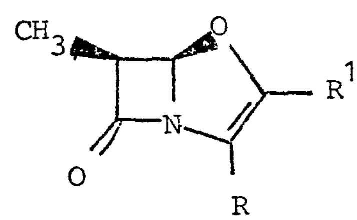

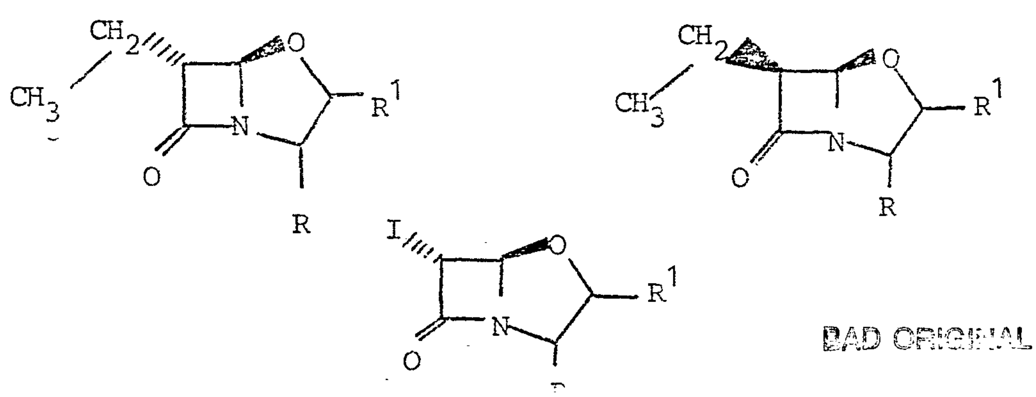

BAD ORIGINAL

In the above compounds R represents a free or esterified carboxyl group especially a free carboxyl group or a p-nitrobenzyloxycarbonyl group and $R^1$ represents $-CH^3$, $-CH_2N_3$, $-CH_2SCN$, $-CH_2NHCOR^{15}$ wherein $R^{15}$ represents a lower alkyl group, $-CH_2OCOR^{15}$, $-CH_2Br$ and $-CH_2SHet$ wherein Het represents an aromatic or non-aromatic heterocyclic group, which is especially as described above.

The compounds of formulae II, IIa and IIIa are also provided by the present invention.

Compounds of formulae III, IVa and VI in which $R^2$ and $R^3$ both represent hydrogen atoms are known, but those compounds of formula III, IVa and VI in which $R^2$ or $R^3$, or $R^2$ and $R^3$ represent other than hydrogen atoms are provided by the present invention.

Compounds of formula IVb in which $R^2$ and $R^3$ both represent hydrogen atoms, one of $R^2$ and $R^3$ represents a hydrogen atom and the other a radical as defined for $R^1$, or both represent radicals as defined for $R^1$ are also provided by the present invention.

The invention further provides compounds of formula IXa with the exception of those having a 1-hydroxy ethyl group at position 6, and the compounds of formula IXc with the exception of those having an ethylidene group at position 6.

All of the compounds that are provided by the invention may exist in any appropriate isomeric form, as discussed above, either as a pure isomer or as a mixture of any two or more isomers.

The present invention also provides the salts of those compounds of the invention that have salt-forming groups, especially the salts of free acids of formula I and the acid addition salts of appropriate compounds of formula I. The salts are especially physiologically tolerable salts, for example, alkali metal and alkaline earth metal salts, ammonium salts and salts with an appropriate organic amine; also physiologically tolerable acid addition salts with suitable inorganic and organic acids, for example, hydrochloric acid, sulphuric acid, carboxylic and organic sulphonic

acids, for example, trifluoroacetic acid and *p*-toluene-sulphonic acid.

A salt of a free acid of formula I may be produced by reacting the free acid with the appropriate base in a solvent, preferably under conditions under which the salt precipitates. In the case of an alkali metal salt, for example, a sodium or potassium salt, the preferred base is an alkoxide.

A salt may be produced directly from an ester by splitting off the ester group under suitable reaction conditions, for example, catalytic reduction of an ester, for example, a *p*-nitrobenzyl ester, in an aqueous/organic solvent, for example, comprising water and ethyl acetate, dioxane, or tetrahydrofuran, in the presence of an appropriate metal salt, especially a bicarbonate, for example, in an equivalent amount or in a slight excess, yields a salt directly.

The compounds of formula I and salts thereof are ß-lactamase inhibitors, and the compounds are generally stable to the action of ß-lactamases produced by gram-positive organisms, for example, by *Staphylococcus aureus* and gram negative organisms, for example, *Enterobacter cloacae*. They also possess antibacterial properties themselves and may be used, for example, to treat bacterial infections in human and other animals.

The invention accordingly provides a compound of formula I whenever obtained according to the process of the present invention, or a compound of formula X, XI, XII or XIII, or a physiologically tolerable salt thereof, or a mixture of two or more such substances as active ingredient, in admixture or conjunction with a pharmaceutically suitable carrier. The preparation may also comprise one or more other pharmaceutically active substances, for example, another antibacterial substance, especially one which has a ß-lactam ring. The preparations may be in a form suitable for enteral or parenteral administration, for example, for oral, intraveneous, or intramuscular administration, for example, as tablets, capsules, syrups, or sterile injectable or infusible solutions. The preparations are advantageously in unit dosage form and preferably comprise from 50 to 100 mg of the active ingredient. The daily dosage of the active substance is generally from 100 to 5,000 mg, in divided doses.

The invention also provides the use of a compound of the invention as a ß-lactamase inhibitor and/or as an antibacterial agent.

The invention also provides a pharmaceutical preparation which comprises a compound of the general formula I or a physiologically tolerable salt thereo or a mixture of two or more such substances, and one or more further pharmaceutically active substances, for example, as described above and, for example, in unit dosage form.

Unit dosages are preferably as described above.

The following Examples 9 to 18 illustrate the invention. In them, temperatures are expressed in degree Celsius and ratios of solvents for chromatography are by volume. Examples 1 to 8 illustrate the preparation of starting materials and intermediates.

## E X A M P L E 1:

## 4-Nitrobenzylpenicillanate (VI)

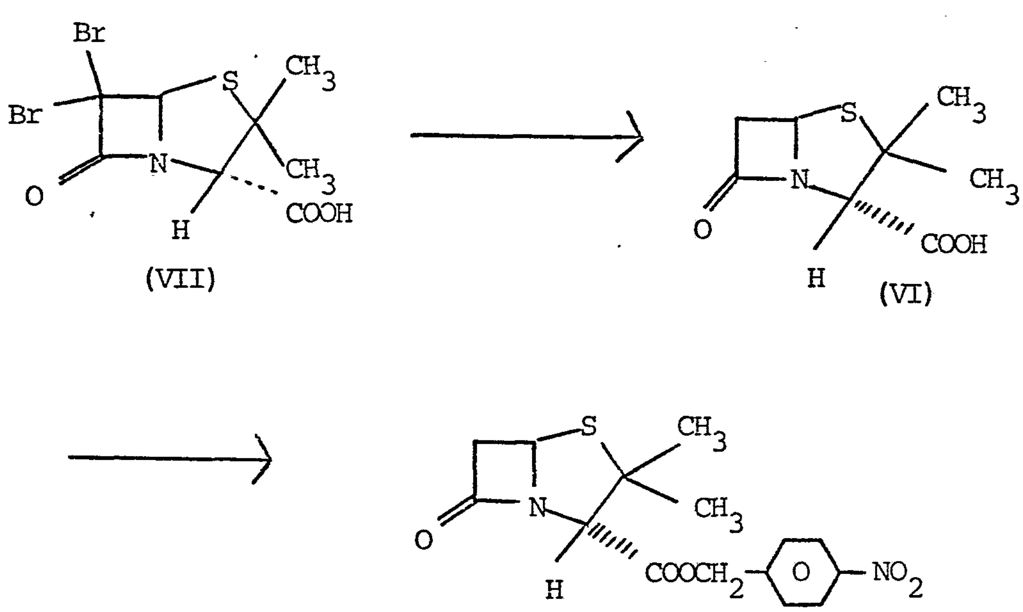

(VII)

(VI)

(a) Penicillanic acid (VI)

16.7 g of 6,6-dibromopenicillanic acid (VII) were taken up in a solution of 3.9 g of sodium bicarbonate in 250 ml of water and the solution was filtered. 8.5 g of 5 % Pd/$CaCO_3$ were added, and the mixture was then hydrogenated at atmospheric pressure with vigorous shaking for 3 hours. TLC analysis (silica gel, chloroform-ether-formic acid 7:2:1) showed the absence of starting material.

The catalyst was removed by filtration through Hyflo Supercel, and the filter bed was washed well with sodium bicarbonate solution (4 x 50 ml). The combined filtrates were acidified to pH 2, and extracted with ether (6 x 100 ml). The combined ether extracts were washed with brine (2 x 200 ml), dried ($MgSO_4$), and evaporated to leave a pale yellow oil (6.9 g, 74 %). This material was unstable and was converted to the ester as soon as possible.

$\delta$ ($CDCl_3$) 1.57 (3H, s, $CH_3$), 1.70 (3H, s, $CH_3$) 3.05 (1H, dd, $J_{5,6\beta}$2Hz, 6ß-H), 3.62 (1H, dd, $J_{5,6\alpha}$ 4Hz, $J_{6\alpha,6\beta}$16Hz, 6α-H) 4.47 (1H, s, 3-H), 5.27 (1H, dd, 5-H), 9.95 (1H, s, -COOH).

(b) 4-Nitrobenzylpenicillanic acid (VI)

To 6.9 g of penicillanic acid (VI) in 70 ml dimethylacetamide at 0$^o$ was added 3.1 g of diisopropylamine. 6.5 g of 4-nitrobenzyl bromide in 20 ml dimethylacetamide was added over 5 minutes and the reaction mixture was stored at 0$^o$ for 18 hours.

The white crystalline precipitate was filtered off and the filtrate poured into water (200 ml) whereupon the mixture was adjusted to pH 2 by addition of hydrochloric acid. The mixture was extracted with ethyl acetate (3 x 100 ml) and the extracts washed with dilute hydrochloric acid (pH 2, 2 x 100 ml), water (4 x 100 ml), sodium bicarbonate (2 x 50 ml), water (2 x 50 ml), and brine (2 x 50 ml). The organic solution

was dried over magnesium sulphate and the solvent evaporated to leave a yellow solid. The solid was triturated with ethyl acetate-ether and dried to leave a white solid (6.2 g). A second crop of crystals yielded further product (0.3 g).
Total yield 6.5 g; 41 % on 6,6-dibromopenillianic acid, m.p. 134° (from diethyl ether)
$\nu$ max ($CCl_4$)1793, 1760 $cm^{-1}$
$\delta$ ($CDCl_3$) 1.43 (3H, s, $CH_3$), 1.67 (3H, s, $CH_3$), 3.05 (1H, dd, $J_{5,6ß}$2Hz, 6ß-H), 3.62 (1H, $J_{5,6\alpha}$4Hz, $J_{6\alpha,6ß}$ 16Hz, 6$\alpha$-H), 4.53 (1H, s, 3-H), 5.27 (3H, m, $-CH_2$, 5-H), 7.4-8.4 (4H, m, $C_6H_4$).
m/e 336 ($M^+$), 114 (base peak)

E X A M P L E 2:
4-Nitrobenzylpenicillanate 1-oxide

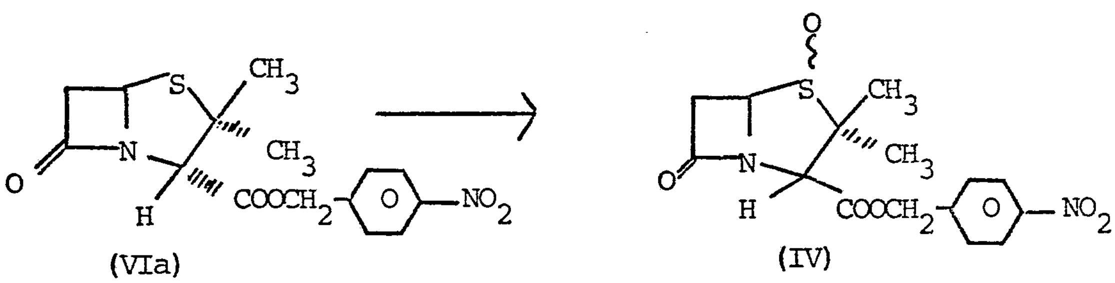
(VIa) (IV)

To 6.1 g of 4-nitrobenzylpenicillanate (VIa) in 100 ml ethyl acetate at 0° was added 3.51 g of 3-chloroperbenzoic acid (85 % pure solid) in 35 ml ethyl acetate dropwise over 20 min keeping the temperature <5°. The reaction was checked for completion by TLC (silica gel, chloroform-ether-formic acid 7:2:1), then the white precipitate was filtered off and washed with ethyl acetate and ether. Yield 2.62 g.

The filtrate was washed with sodium bicarbonate and brine, dried ($MgSO_4$) and the solvent evaporated. Trituration with ether gave 2.94 g of a white solid, shown to be a mixture of $\alpha$(R) and ß(S) sulphoxides, with the ß-sulphoxide predominating.
Total yield 5.65 g, 87 %, m.p. 147° (from ethyl acetate)

$\nu_{max}$ ($CHCl_3$) 1793, 1757 $cm^{-1}$
$\delta$ ($CDCl_3$) <u>ß-sulphoxide</u> 1.15 (3H, s, $\alpha$-$CH_3$), 1.67 (3H, s, ß-$CH_3$, 3.33 (2H, d, $J_{5,6}$ 3Hz, $6\alpha$, $6_\beta$-H), 4.45 (1H, s, 3-H), 4.9 (1H, t, 5-H), 5.25 (2H, s, -$CH_2$-), 7.3-8.2 (4H m, $C_6H_4$)
<u>$\alpha$-sulphoxide</u> 1.33 (s, $\alpha$-$CH_3$), 1.55 (s, ß-$CH_3$), 3.55 (m, $6\alpha$,$6_\beta$-H), 4.75 (m, 5-H)
m/e 352 ($M^+$), 336 (M-O), 136 (base peak, -$CH_2PhNO_2$).

<u>E X A M P L E 3</u>:

<u>4-Nitrobenzyl-2-/4R-(2-benzthiazolyl-dithio)-2-oxo-1-azetidinyl7-2-(1-propen-2-yl)-acetate</u>

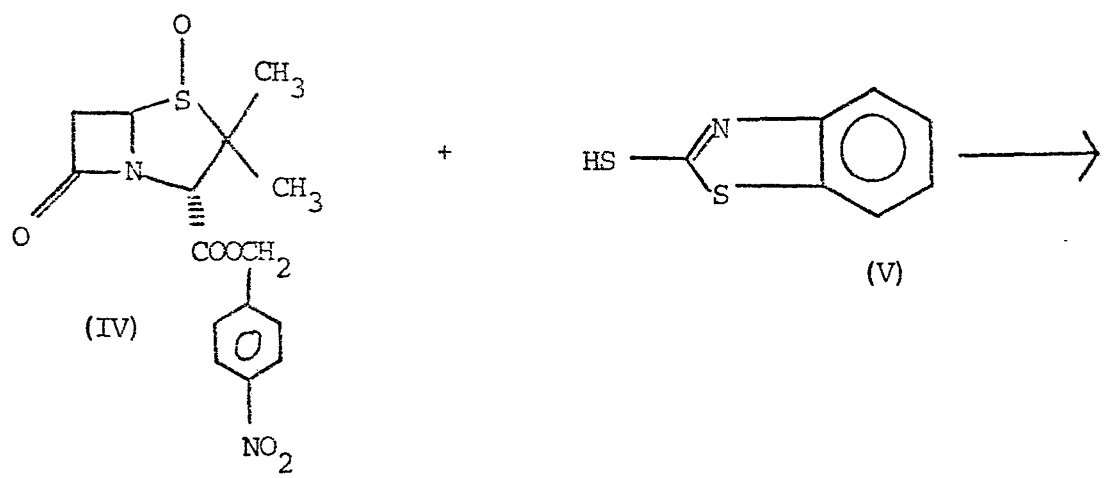

(IV)

(V)

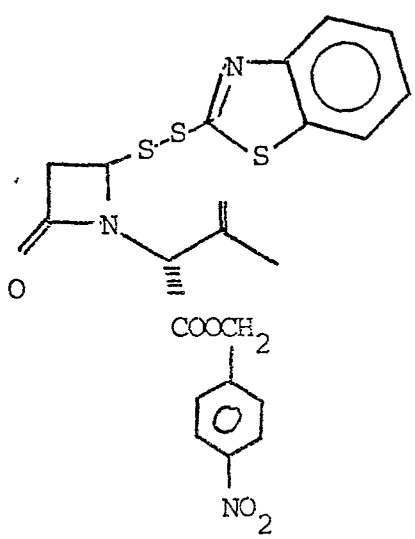

(III)

14.1 g of 4-nitrobenzylpenicillanate 1-oxide (IV) and 6.84 g of 2-mercaptobenzothiazole (V) (98 % pure) were heated for about 6 hours in refluxing toluene (250 ml) under nitrogen until TLC analysis (ethyl acetate-cyclohexane 1:2) showed reaction was complete.

The cold reaction mixture was washed with saturated sodium carbonate (1 x 40 ml), water (5 x 50 ml) and brine 2 x 50 ml), dried ($MgSO_4$) and the solvent evaporated. The product was triturated with ethyl acetate-ether to give a pale yellow solid which was filtered off and dried *in vacuo*. Yield 13.7 g (68 %).

m.p. 121° (from ethyl acetate).

$\nu_{max}$ ($CH_2Cl_2$) 1778, 1748 $cm^{-1}$

$\delta$ ($CDCl_3$) 1.94 (3H, S, $CH_3$), 3.4 (2H, m, 3-H), 4.88, 5.02 (2H, 2s, $=CH_2$), 5.2 (4H, m, $-CH_2-$, 2'-H, 4'-H), 7.2-8.4 (8H, m, $C_6H_4$)

m/e 334 (M-167), 303 (M-167-S), 166 (base peak)

| | | | |
|---|---|---|---|
| Found: | C 52.5 %; | H 3.8 %; | N 8.5 % |
| Calculated: | C 52.7 %; | H 3.8 %; | N 8.4 %. |

E X A M P L E 4:

4-Nitrobenzyl-2-[4S-(2-benzthiazolyl-thio)-2-oxo-1-azetidinyl]-2-(1-propen-2-yl)-acetate (IIIa)

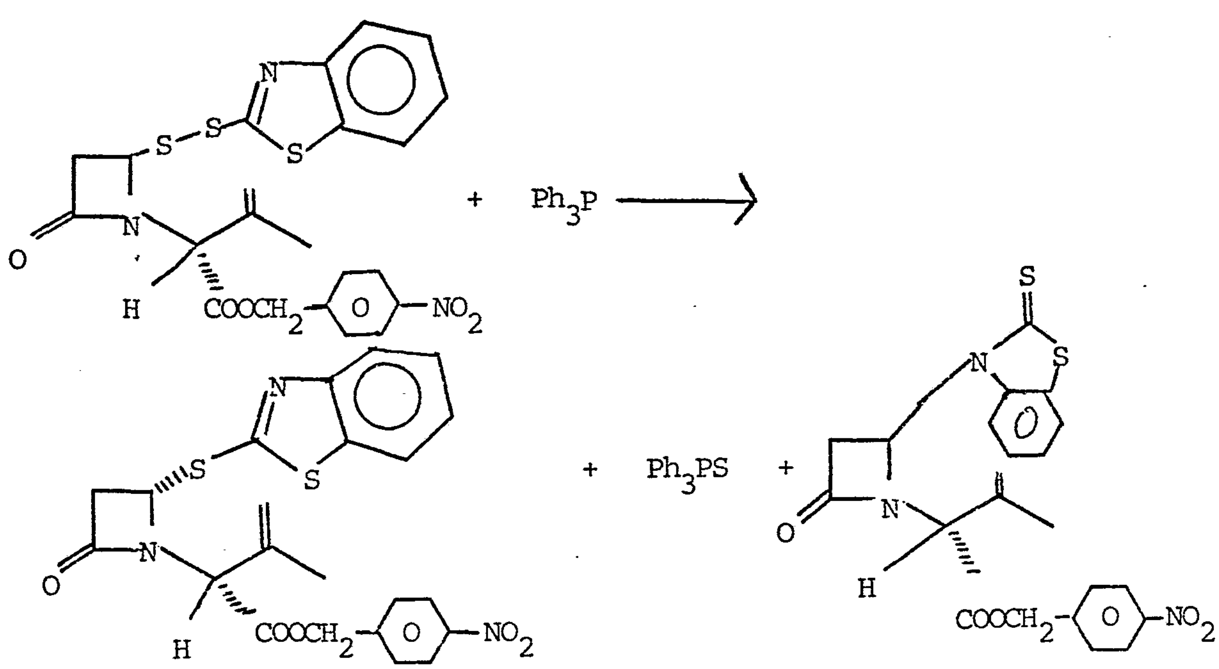

To 1.0 g of 4-nitrobenzyl-2-[4R-(2-benzthiazolyldithio)-2-oxo-1-azetidinyl]-2-(1-propen-2-yl)-acetate (III) in 3 ml dichloromethane at room temperature was added, with stirring, 0.55 g of triphenylphosphine. The mixture was stirred for 30 minutes then diluted with an equal volume of hexane. The total solution was chromatographed on silica gel, using a dichloromethane-hexane mixture as eluent. Excess triphenylphosphine and triphenylphosphine sulphide eluted first. The eluent was then changed to dichloromethane and compound IIIa (4 parts) and its N-bonded isomer (compound IX) (1 part) eluted as a mixture. Fractions containing compounds IIIa and IX were combined and solvent evaporated to give a foam (0.675 mg), which was recrystallized from ethyl acetate-cyclohexane (1:1 v/v) to give a white solid (0.38 g, 41 %) m.p. 110°. By NMR analysis the solid was shown to be the pure compound IIIa. The stereochemistry at $C_3$ was established by further experiments.

More extensive chromatography of the mixture of compounds IIIa and IX gave pure compound IX as an oil.

4-Nitrobenzyl-2-[4S-(2-benzthiazolyl-thio)-2-oxo-1-azetidinyl]-2-(1-propen-2-yl) acetate (IIIa)

---

$\delta$ ($CDCl_3$) 1.92 (3H, s, $CH_3$), 3.18 (1H, dd, $J_{3\alpha,4}$ 2.5Hz, $3_\alpha$-H) 3.69 (1H, dd, $J_{3\beta,4}$ 5Hz, $J_{3\alpha,3\beta}$ 15Hz, 3β-H), 5.0 (3H, m, $=CH_2$, 2-H), 5.28 (2H, s, $-CH_2-$), 5.78 (1H, dd, $4_\beta$-H), 7.2-8.4 (4H, m, $C_6H_4$).

$\nu_{max}$ ($CCl_4$) 1784, 1755 $cm^{-1}$

m/e 469 ($M^+$), 303 (M- -S- 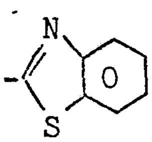, 122 (M- -S- 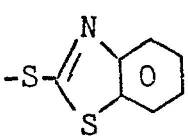, - 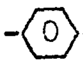 -$NO_2$), 136 (base peak) $-CH_2-$ 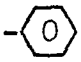 -$NO_2$.

Found: C 56.1; H 4.0; N 9.1 %; m/e 469.0741

Calculated: C 56.3; H 4.1; N 9.0 %; m/e 469.0767

E X A M P L E 5:

4-Nitrobenzyl-2-/4S-(2-benzthiazolyl-thio)-2-oxo-1-azetidinyl7-3-hydroxycrotonate (IIa)

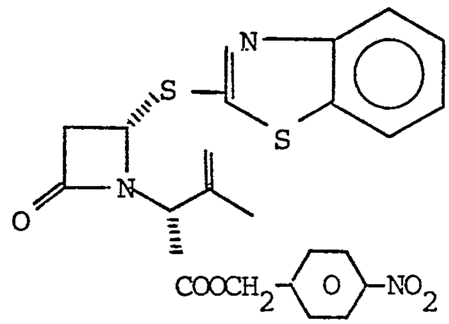

(IIIa)

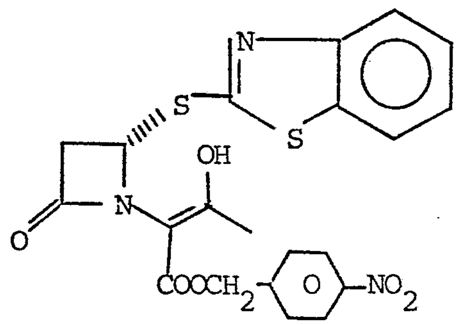

(IIa)

1.28 g of 4-Nitrobenzyl-2-/4S-(2-benzthiazolylthio)-2-oxo-1-azetidinyl7-2-(1-propen-2-yl)-acetate (IIIa) in 30 ml dry dichloromethane at -10$^{o}$ was treated with an air-ozone mixture until TLC analysis (silica gel, ethyl acetate-cyclohexane 1:1), showed the absence of starting material. The solution was then flushed with air followed by the addition of 1.68 g of dimethyl sulphide, and the mixture stirred for 1 hour. The solvent was then evaporated off and the oily residue was chromatographed on silica gel using dichloromethane-ethyl acetate (9:1) as eluent. Fractions containing the enol were combined and evaporated to give the product as as a white foam.

$\delta$ ($CDCl_3$) 2.17 (3H, s, $-CH_3$), 3.17 (H, dd, $J_{trans}$ 3Hz 3$\alpha$-H) 3.69 (1H, dd, $J_{cis}$ 6Hz, $J_{gem}$ 15 Hz, 3ß-H), 5.33 (2H, s, $-CH_2-$), 6.05 (1H, dd, 4-H), 7.3-8.4 (8H, m, $C_6H_4$).
m/e 471 ($M^+$), 277, 262, 167 (base peak).

Found: m/e 471.0499 $C_{21}H_{17}N_3O_6S_2$ requires m/e 471.0559

E X A M P L E 6:

4-Nitrobenzyl-2-/4R-(2-benzthiazolyl-dithio)-2-oxa-1-azetidinyl7-3-hydroxy-crotonate (II)

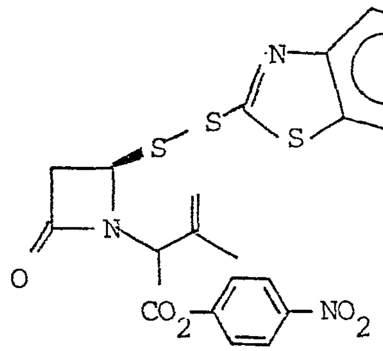
(III)

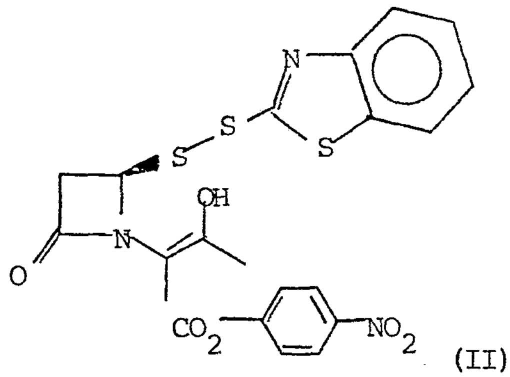
(II)

2.0 g of 4-nitrobenzyl-2-[4R-(2-benzthiazolyldithio)-2-oxo-1-azetidinyl]-2-(1-propen-2-yl)-acetate (III), in a mixture of 45 ml dry dichloromethane and 15 ml dry methanol were cooled to a temperature of from -50° to -78° in a dry ice-acetone bath. An air-ozone mixture was passed through the solution until the analysis (silica gel, ethyl acetate-hexane 1:1) indicated the absence of starting material. 2.0 g of dimethyl sulphide were added at -78° and the solution allowed to warm to room temperature. The solvent was evaporated *in vacuo* and the residue chromatographed over silica gel using ethylacetate-hexane mixtures as eluent to give pure enol 1.72 g 86 % as a colourless foam.

$\nu_{max}$ 1780 $cm^{-1}$

$\delta$ ($CDCl_3$) 2.15 (3H, s, $CH_3$), 3.1 (1H, dd, $J_{3\beta,4}$ 2Hz, 3ß-H), 3.5 (1H, dd, $J_{3\alpha,4}$ 4Hz, $J_{3\alpha,3\beta}$ 16Hz, $3_{\alpha-H}$), 5.0-5.35 (3H, m, $-CH_2$, 4-H), 7.0-8.4 (8H, m, $C_6H_4$), 12.8 (1H, s, -OH)

m/e 265, 200, 167 (base peak, 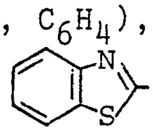-SH), 167 ($-CH_2C_6H_4NO_2$)

Found: C 50.0; H 3.6; N 8.2

Calculated: C 50.1; H 3.40; N 8.3.

E X A M P L E 7:

4-Nitrobenzyl-2-[4R-(2-pyridyl-dithio)-2-oxo-1-azetidinyl]-2-(1-propen-2-yl)-acetate (III)

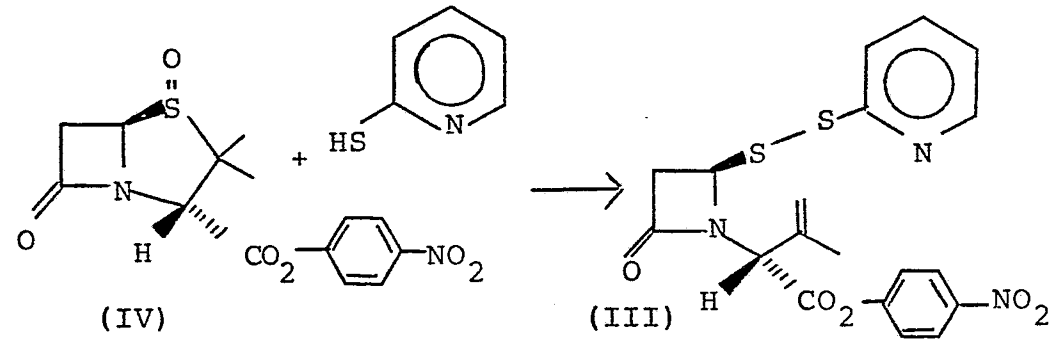

(IV) (III)

To a solution of 2 g of 4-nitrobenzylpenicillanate 1-oxide (IV) in 40 ml dry toluene was added 0.63 g 2-mercaptopyridine. The mixture was refluxed under nitrogen for 1.5 hours. The solvent was then evaporated off <u>in vacuo</u>, and the residue was chroamtographed on silica gel, eluting with ethyl acetate-hexane to yield pure disulphide 1.932 g (76 %) as a colourless syrup.

$\gamma_{max}$ ($CHCl_3$) 1770, 1750 $cm^{-1}$

$\delta$ ($CDCl_3$) 1.95 (3H, bs, $CH_3$) 3.11 (1H, dd $J_{3\beta,3\alpha}$ 16Hz, $J_{3\beta,4}$ 3Hz, 3-H) 3.44 (1H,dd, $J_{3\alpha,3\beta}$ 16Hz, $J_{3\alpha,4}$ 4Hz, 3-H) 4.87 (1H, s, 2'-H), 5.02-5.29 (3H, m, 4-H, $=CH_2$) 5.35 (2H, s, $-CH_2-$) 7.06-8.70 (8H, m, aromatics).

E X A M P L E 8:

4-Nitrobenzyl-2-/4R-(2-pyridyl-dithio)-2-oxo-1-azetidinyl7-3-hydroxy-crotonate (II)

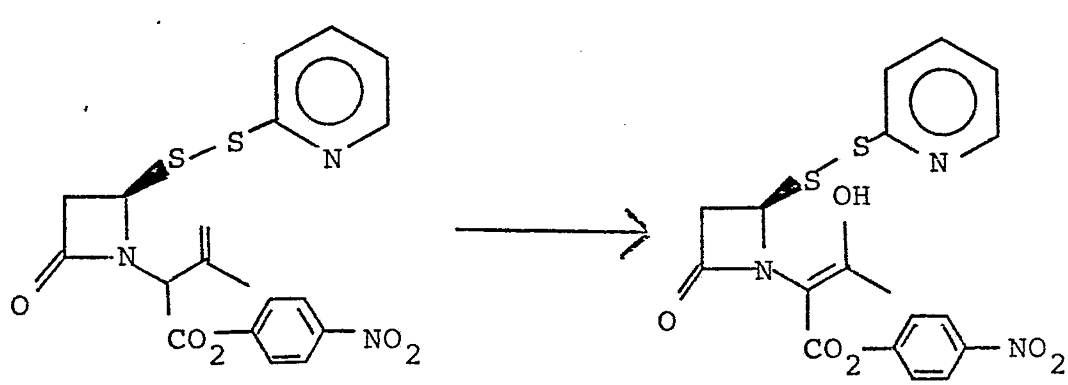

(III) (II)

0.65 g of 4-nitrobenzyl-2-/4R-(2-pydridyldithio)-2-oxo-1-azetidinyl7-2-(1-propen-2-yl)-acetate in 10 ml dry dichloromethane at -78° were treated with an air-ozone mixture until TLC indicated complete loss of starting material (silica gel, ethyl acetate-hexane). 0.25 ml methyl sulphide were added at -78° and the solution was allowed to warm to room temperature. The solution was evaporated in vacuo and the residue was chromatographed on silica gel eluting with ethyl acetate-hexane to give the pure crotonate II (0.356 g, 54.8 %) as a colourless oil.

$\nu_{max.}$ 1770, 1665 $cm^{-1}$

$\delta$ ($CDCl_3$) 2.16 (3H, s, $CH_3$) 3.03 (1H, dd $J_{3\alpha,3\beta}$ 15HZ, $J_{3\beta,4}$ 3Hz, 3-H), 3.43 (1H, dd $J_{3\alpha,3\beta}$ 15Hz, $J_{3\alpha,4}$ 5Hz, 3-H), 5.14 (1H, dd J 3Hz, 5 Hz, 4-H), 5.15 (1H, d, J 13 Hz, $-CH_2-$), 5.43 (1H, d J 13 Hz, $-CH_2-$) 7.00-8.63 (8H, m, aromatics). 12.22 (1H, s, -OH).

E X A M P L E 9:

2-Methyl-3-(4-nitrobenzyloxycarbonyl)-oxapenem (I)

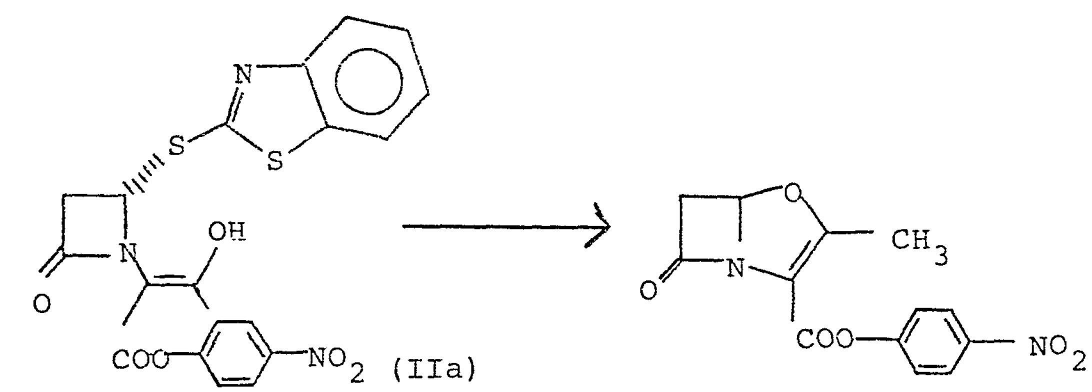

(IIa)

(I)

To 35 mg of 4-nitrobenzyl-2-/4S-benzthiazolylthio-2-oxo-1-azetidinyl7-3-hydroxycrotonate (IIa) in 0.7 ml $CDCl_3$ was added 6.4 µl pyridine followed by 25 mg of silver methylsulphonate and the mixture was stirred for 20 minutes at room temperature, then the reaction mixture was centrifuged and the supernatant was examined by NMR spectroscopy and TLC. Both analyses clearly indicated the presence of the oxapenem I, by comparison with a known sample.

BAD ORIGINAL

E X A M P L E 10:

2-Methyl-3-(4-nitrobenzyloxycarbonyl)-oxapenem (I)

The procedure of Example 9 was carried out except that 6.5 µl of pyridine were used, and 38.1 mg of silver trifluoromethylsulphonate was used instead of the silver methylsulphonate. Both NMR and TLC analyses indicated the presence of the desired oxapenem.

E X A M P L E 11:

2-Methyl-3-(4-nitrobenzyloxycarbonyl)-oxapenem

To a stirred solution of 0.338 g of silver carbonate and 0.428 g of triphenylphosphine in 4 ml dry chloroform was added dropwise a solution of 0.80 g of 4-nitrobenzyl-2-/4R-benzthiazolyldithio-2-oxo-1-azetidinyl/-3-hydroxy-crotonate in dry chloroform. The resulting mixture was stirred at room temperature for half an hour and then chromatographed directly (short path rapid chromatography, silica gel H, methylene chloride/hexane) to give 0.195 g (41 % of the theoretical yield) of 2-methyl-3-(4-nitrobenzyloxycarbonyl)-oxapenem as a colourless, crystalline solid.

$\delta$ ($CDCl_3$) 2.32 (3H, s, $CH_3$) 3.42 (1H, dd $J_{gem}$ 17Hz $J_{trans}$ 1Hz, 6-H) 3.86 (1H, dd $J_{gem}$ 17 Hz $J_{cis}$ 2.5 Hz, 6-H) 5.22 (1H, AB d J 14 Hz, $-CH_2-$) 5.50 (1H, AB d J 14Hz, $-CH_2-$) 5.93 (1H, dd $J_{trans}$ 1Hz $J_{cis}$ 2.5Hz, 5-H), $\nu_{max}$ = 1810, 1710, 1640 $cm^{-1}$, mp 128.5 - 129°C.

E X A M P L E 12:

2-Methyl-3-(4-nitrobenzyloxycarbonyl)-oxapenem

To a suspension of 0.024 g of silver oxide and 0.028 g of triphenylphosphine in 0.2 ml deuterochloroform was added dropwise a solution of 0.050 g of 4-nitrobenzyl-2-/4R-benzthiazolyldithio-2-oxo-1-azetidinyl/-3-hydroxy-crotonate in 0.4 ml deuterochloroform. After 10 minutes the reaction mixture was centrifuged, and NMR spectroscopic examination of the supernatant confirmed the presence of 2-methyl-3-(4-nitrobenzyloxycarbonyl)-oxapenem as the main product.

EXAMPLE 13:

2-Methyl-3-(4-nitrobenzyloxycarbonyl)-oxapenem

To a stirred solution of 0.05 g of 4-nitrobenzyl-2-[4R-benzthiazolyldithio-2-oxo-1-azetidinyl]-3-hydroxy-crotonate and 0.0134 g of copper acetylacetonate in 0.4 ml deuterochloroform was added 0.0267 g of triphenylphosphine in 0.1 ml deuterochloroform. After 5 minutes the solution was centrifuged. NMR spectroscopy of the solution showed 2-methyl-3-(4-nitrobenzyloxycarbonyl)-oxapenem as the major product. TLC analysis confirmed this result.

EXAMPLE 14:

2-Methyl-3-(4-nitrobenzyloxycarbonyl)-oxapenem

A solution of 0.05 g of 5-nitrobenzyl-2-[4R-benzthiazolyldithio-2-oxo-1-azetidinyl]-3-hydroxy-crotonate and 10 µl deuteropyridine in 0.5 ml deuterochloroform was added to a stirred solution of 0.028 g of triphenylphosphine and 0.0201 g of silver methane-sulphonate in 0.3 ml deuterochloroform. After 5 minutes the reaction mixture was centrifuged to remove the precipitated solid, and NMR spectroscopy of the supernatant showed that 2-methyl-3-(4-nitrobenzyloxycarbonyl)-oxapenem was the major product. TLC analysis confirmed this result.

EXAMPLE 15:

2-Methyl-3-(4-nitrobenzyloxycarbonyl)-oxapenem

To a solution of 0.05 g of 4-nitrobenzyl-2-[4R-benzthiazolyldithio-2-oxo-1-azetidinyl]-3-hydroxy-crotonate in 0.5 ml deuterochloroform was added 0.028 g of triphenylphosphine in 0.1 ml deuteriochloroform. The solution was shaken at room temperature. NMR indicated the formation of 2-methyl-3-(4-nitrobenzyloxycarbonyl)-oxapenem and 4-nitrobenzyl-2-[4S-benzthiazolylthio-2-oxo-1-azetidinyl]-3-hydroxy-crotonate in the ratio 1:4.

EXAMPLE 16:

2-Methyl-3-(4-nitrobenzyloxycarbonyl)-oxapenem

To a solution of 0.075 g of 4-nitrobenzyl-2-[4-pyridine-

dithio-2-oxo-1-azetidinyl7-3-hydroxy-crotonate in 4 ml dry methylene chloride was added 0.045 g of triphenylphosphine in 1 ml dry methylene chloride. The solution was stirred for 1.5 hour, evaporated in vacuo, and NMR analysis of the residue showed the formation of 2-methyl-3-(4-nitrobenzyl-carbonyl)-oxapenem and 4-nitrobenzyl-2-/4-pydridinethio-2-oxo-1-azetidinyl7-3-hydroxy-crotonate in the ratio 1:1. The residue was then chromatographed on silica gel (2 g) eluting with ethyl acetate-hexane mixtures to afford triphenyl phosphine sulphide then 10 mg of 2-methyl-3-(4-nitrobenzyloxy)-carbonyl-oxapenem as a colourless oil which crystallised on standing.
$\nu$ max 1810 $cm^{-1}$ mp 128.5 - 129°C.

E X A M P L E 17:

2-Methyl-3-(4-nitrobenzyloxycarbonyl)-oxapenem

To 33 mg of 4-nitrobenzyl-2-/4S-benzthiazolylthio-2-oxo-1-azetidinyl7-3-hydroxycrotonate in 0.5 ml of chloroform was added, at room temperature, excess sodium hydride, followed by 25 mg of silver trifluoromethylsulphate, and the mixture was stirred for 6 1/2 hours. The solid by-products were filtered off and the solvent was evaporated to give a crystalline solid, shown by TLC and NMR analysis to be 2-methyl-3-(4-nitrobenzyloxycarbonyl)-oxapenem.

E X A M P L E 18:

2-Methyl-3-(4-nitrobenzyloxycarbonyl)-oxapenem

To 200 mg of 4-nitrobenzyl-2-/4S-benzthiazolylthio-2-oxo-1-azetidinyl7-3-hydroxy-crotonate in 5 ml of dry chloroform was added, with stirring, 47 mg of triethylamine. After 1 minute, 220 mg of freshly ground silver trifluoromethylsulphonate was added at room temperature, and the mixture was stirred for 15 minutes. The precipitate was removed by centrifugation and the solution applied to a short-path silica-gel column. Rapid elution with dichloromethane-hexane gave fractions containing 2-methyl-3-(4-nitrobenzyloxycarbonyl)-oxapenem. These fractions were combined and evaporated under reduced pressure to give 27 mg of the product as a white solid.

EXAMPLE 19:

Sodium salt of 2-methyloxapenem

To a solution of 0.10 g of 2-methyl-3-(4-nitrobenzyloxycarbonyl)-oxapenem in 7 ml ethyl acetate was added 0.10 g of palladium on charcoal (10 % by weight) and a solution of 0.027 g of sodium bicarbonate in 7 ml of water. The reaction mixture was hydrogenated until the reaction was judged complete (TLC), then the aqueous solution washed with ethyl acetate then lyophilized to afford the sodium salt of 2-methyloxapenem.

EXAMPLE 20:

4-Nitrobenzyl 6(R,S)-(phenylhydroxymethyl)-penicillanate

(4-Nitrobenzyl 5(R)-3,3-dimethyl-6(R,S)-(phenylhydroxymethyl)-7-oxo-4-thia-1-azabicyclo/3,2,0/heptane-2(R)-carboxylate)

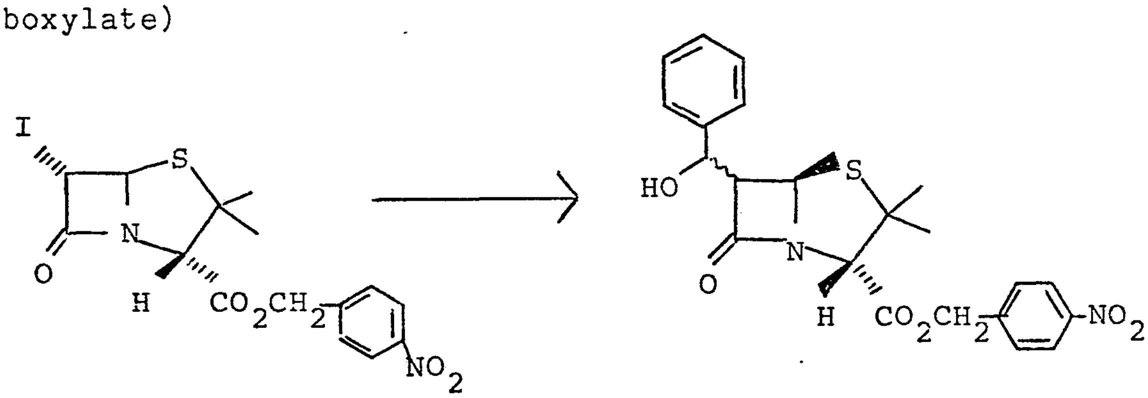

Under an argon atmosphere, a stirred solution of 1.61 g of 4-nitrobenzyl 6(S)-iodopenicillanate in 30 ml of dried tetrahydrofuran was cooled to -110° and then treated with 2.0 ml of a 3M ethereal solution of methylmagnesium bromide. After the solution had been stirred for 5 minutes, 3.6 ml of benzaldehyde was added. The solution was allowed to warm to -40° over 30 minutes, was quenched by addition of 10 ml of saturated aqueous ammonium chloride and was then poured into 0.01M HCl. The pH of the solution was

readjusted to 2.0 with 2M HCl, and the mixture was then extracted with ethyl acetate (3 x 25 ml). The combined organic extracts were backwashed with water and brine, and were dried over sodium sulphate. Evaporation in vacuo yielded an oil which was chromatographed over silica gel. Elution with cyclohexane:ethyl acetate (2:1) yielded the 6(R) alcohol (109 mg).

$\delta$ ($CDCl_3$) 1.38 (3H, s, $\alpha$-$CH_3$), 1.72 (3H, s, $\beta$-$CH_3$), 3.0 (H, br, OH), 3.93 (1H, dd, J 4Hz; J 9 Hz, 6-H), 4.50 (1H, s, 3-H), 5.10 (2H, m and d J 4Hz, $\gamma$-H and 5-H), 5.23 (2H, s, $CH_2$), 7.3-8.3 (9H, m, aromatics); and the 6(S) alcohol (288 mg).

$\delta$ ($CDCl_3$) 1.37 (3H, s, $\alpha$-$CH_3$), 1.58 (3H, s, $\beta$-$CH_3$), 3.3 (1H, br, OH), 3.67 (1H, dd J 2Hz, J 5Hz, 6-H), 4.50 (1H, s, 3-H), 5.05 (1H, d, J 5Hz, $\gamma$ -H), 5.20 (3H, br, $CH_2$ and 5-H), 7.3-8.3 (9H, m, aromatics).

EXAMPLE 21:

4-Nitrobenzyl 6(R,S)-(2-hydroxy-2-propyl)-penicillanate

(4-Nitrobenzyl 5(R)-3,3-dimethyl-6(R,S)-(2-hydroxy-2-propyl)-7-oxo-4-thia-1-azabicyclo/3,2,0/heptane-2(R)-carboxylate)

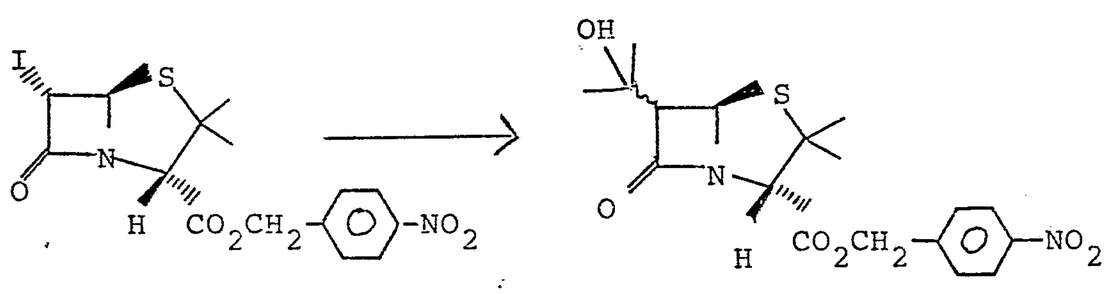

Under an argon atmosphere a stirred solution of 540 mg of 4-nitrobenzyl 6(S)-iodopenicillanate in 11 ml of tetrahydrofuran was cooled to -100°, and then treated with an ethereal solution of 2.0 mmol of methylmagnesium bromide. After the solution had been stirred for 5 minutes, 0.5 ml of acetone was added. The solution was then allowed to

warm to -40° over 30 minutes, was quenched by addition of 5 ml of saturated ammonium chloride and was poured into 100 ml of 0.01M HCl. The pH of the solution was readjusted to 2.0 with 2M HCl, and the mixture was then extracted with ethyl acetate (3 x 25 ml). The combined organic extracts were backwashed with water and brine, and then were dried over sodium sulphate. Evaporation *in vacuo* yielded an orange oil (380 mg) which was chromatographed over silica gel. Elution with hexane:ethyl acetate (2:1) afforded as a colourless oil (198 mg, 43 %), a 4:1 6(S/R) mixture of the penicillanate shown above.

δ ($CDCl_3$) 1.33 (3H, s, $CH_3$) 1.42 (6H, s, $CH_3$ and α-$CH_3$), 1.67 (3H, s, ß-$CH_3$), 2.53 (1H, broad, OH), 3.40 (0.8H, d, J 1.5Hz, 6-H), 3.72 (0.2H, d J 5Hz, 6-H), 4.57 (1H, s, 3-H), 5.30 (2.8H, s, $CH_2$ and 5-H), 5.48 (0.2 H, d J 5Hz, 5-H), 7.5-8.4 (4H, m, p-$C_6H_4$).

E X A M P L E 22:

4-Nitrobenzyl 6(S)-(2-hydroxy-2-propyl)-penicillanate-1(S)-oxide

(4-Nitrobenzyl 5(R)-3,3-dimethyl-6(S)-(2-hydroxy-2-propyl)-7-oxo-4-thia-1-azabicyclo/3,2,0/heptane-2(R)-carboxylate-4(S)-oxide)

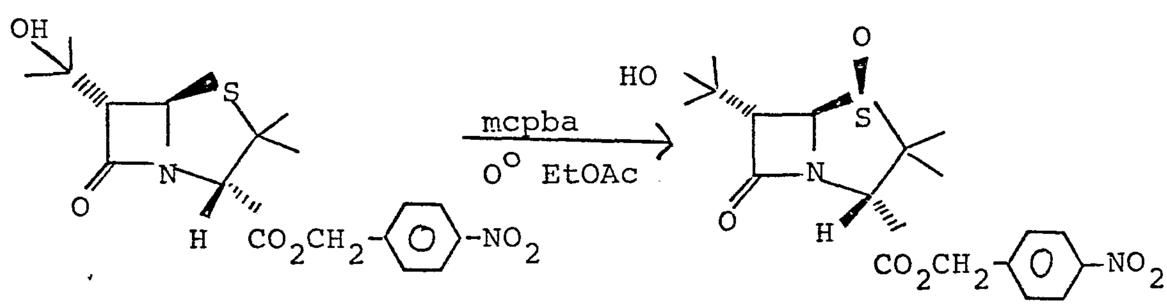


To 1.06 g of 4-nitrobenzyl 6(S)-(2-hydroxy-2-propyl)-penicillanate in 15 ml of ethyl acetate at 0° was added 550 mg of 5-chloroperbenzoic acid (85 % pure solid) in 15 ml of ethyl acetate over 20 minutes, keeping the temperature below 1°. The reaction was checked for completion by

TLC (silica gel, cyclohexane:ethyl acetate (1:2); the solution was evaporated to dryness, and the residue chromatographed over silica gel. Elution with ethyl acetate:hexane (3:1) afforded the above sulphoxide (948 mg, 86 %) as a pale yellow foam.

δ ($CDCl_3$) 1.18 (3H, s, α-$CH_3$), 1.36 (3H, s, $CH_3$), 1.48 (3H, s, $CH_3$), 1.67 (3H, s, ß-$CH_3$), 2.2 (1H, br, OH), 3.62 (1H, d J 2Hz, 6-H), 4.52 (1H, s, 3-H), 4.9 (1H, d J 2Hz, 5-H), 5.25 (2H, z, $CH_2$), 7.2-8.3 (4H, m, p-$C_6H_4$).

E X A M P L E 23:

4-Nitrobenzyl-2-/4(R)-(2-benzothiazoyldithio)-2-oxo-3-(2-hydroxy-2-propyl)-1-azetidinyl7-3-methyl-3-butenate

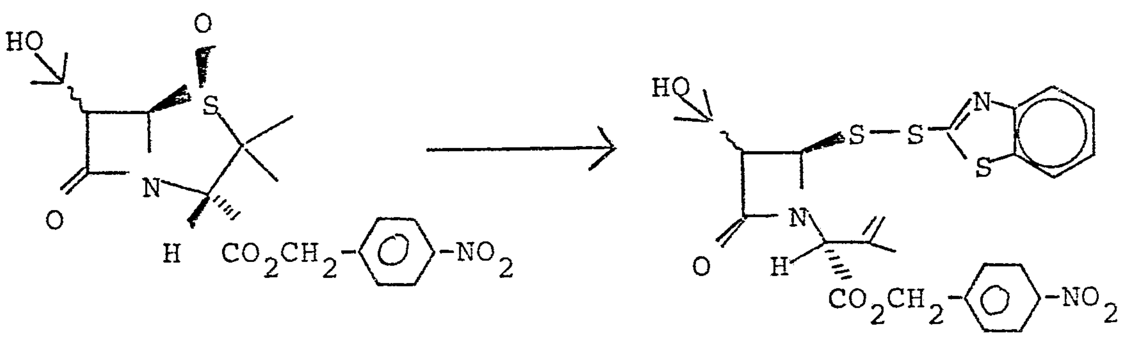

A mixture of 932 mg of 4-nitrobenzyl 6(S)-(2-hydroxy-2-propyl)-penicillanate-1(S)-oxide and 380 mg of 2-mercaptobenzothiazole in 100 ml of toluene was heated in an argon atmosphere under reflux for 12 1/2 hours.

The solution was washed with cold 2M sodium carbonate (2 x 100 ml) and with brine (100 ml), was dried over magnesium sulphate and was evaporated to dryness to yield the above disulphide butenate as a pale yellow foam (1.07 g, 70 %).

δ ($CDCl_3$) 1.38 and 1.43 (6H, s+s, $(CH_3)_2$), 1.95 (3H, br, s, $CH_3$-C=), 2.9 (1H, br, OH), 3.52 (1H, d J 2Hz, 3-H), 4.85 and 5.07 (2H, br s+s, $CH_2$=C), 5.2 (4H, m, $CH_2$ and 2-H and 4'-H), 7.3-8.3 (8H, m, aromatic).

E X A M P L E 24:

4-Nitrobenzyl 2-/4(R)-(2-benzothiazolyldithio)-3-(2-hydroxy-2-propyl)-2-oxo-1-azetidinyl7-3-hydroxycrotonate

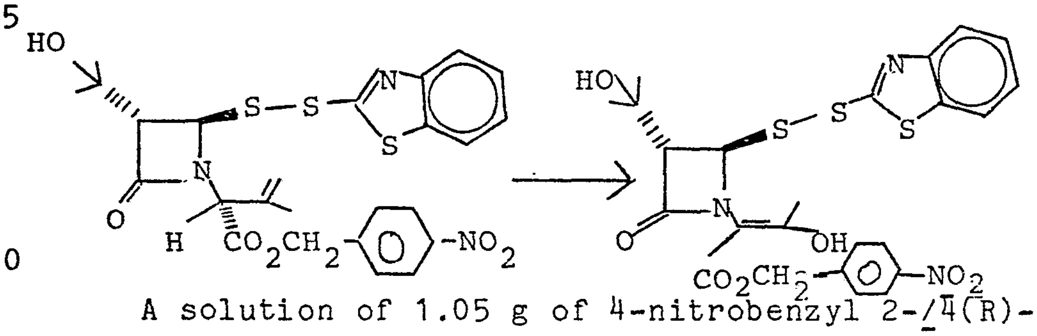

A solution of 1.05 g of 4-nitrobenzyl 2-/4(R)-(2-benzothiazolyldithio)-3(S)-(2-hydroxy-2-propyl)-2-oxo-1-azetidinyl7-3-methyl-butenate in a mixture of 60 ml of dichloromethane and 20 ml of methanol was treated at -78° with an oxygen-ozone mixture until a blue colouration was observed. 1.4 ml of dimethyl sulphide was added, and the solution was allowed to warm to room temperature. After evaporation of the solvent in vacuo, the residue (1.3 g) was dissolved in 100 ml of ethyl acetate, and was washed with water (2 x 100 ml), brine (100 ml), and then dried over magnesium sulphate. Evaporation in vacuo afforded a yellow oil, which was chromatographed over silica gel; elution with hexane: ethyl acetate (2:1) afforded the above enol (569 mg, 51 %).

δ ($CDCl_3$) 1.37 and 1.42 (6H, s+s, $(CH_3)_2C$), 2.17 (3H, s, $CH_3$), 2.3 (1H, bs, OH), 3.42 (0.8H, d J 2Jz, 3'-H), 3.57 (0.2H, d J 5Hz, 3'-H), 5.0-5.3 (3H, m, $CH_2$ and 4'-H), 7.3-8.2 (3H, m, aromatic), 12-13 (0.2H, br, s, =COH), 12.28 (0.8H, br s, =C-OH).

E X A M P L E 25:

6-(2-Hydroxy-2-propyl)-2-methyl-3-(4-nitrobenzyloxycarbonyl)-oxapenem

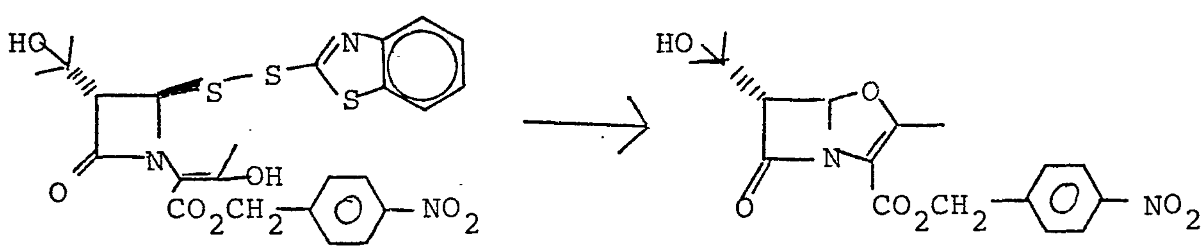

To 206 mg of silver carbonate and 273 mg of triphenylphosphine in 6 ml of chloroform was added a solution of 563 mg of 4-nitrobenzyl-2-/4(R)-(2-benzothiazolyldithio)-3-(2-hydroxy-2-propyl)-2-oxo-1-azetidinyl7-3-hydroxycrotonate in 7 ml of chloroform. After the mixture had been stirred for 10 minutes, it was filtered through Hyflosupercel and evaporated to dryness. The residue was chromatographed on silica gel elution with hexane:ethyl acetate (2:1 changing to 0:1) afforded the above oxapenem ester as a colourless oil (145 mg, 40 %).

$\nu_{max}$ 1745, 1805 $cm^{-1}$

$\delta$ ($CDCl_3$) 1.78 and 1.86 (6H, s+s, $(CH_3)_2$), 2.26 (3H, s, $CH_3$), 3.45 (~ 0.2H, d J 3Hz, 6-H), 3.63 (~ 0.8H, d J ~ 0.5Hz, 6-H), 5.26 (2H, m, $CH_2$), 5.82 (~ 0.8H, d J ~ 0.5Hz, 5-H), 5.93 (~ 0.2H, d J 3Hz, 5-H), 7.3-8.3 (4H, m, p-$C_6H_4$); spectrum from batch 5/145.

E X A M P L E 26:

4-Nitrobenzyl 6(R,S)-(phenylhydroxymethyl)-penicillanate-1(S)-oxide

(4-Nitrobenzyl 5(R)-3,3-dimethyl-6(R,S)-phenylhydroxymethyl)-7-oxo-4-thia-1-azabicyclo/3,2,07heptane-2(R)-carboxylate-4(S)-oxide)

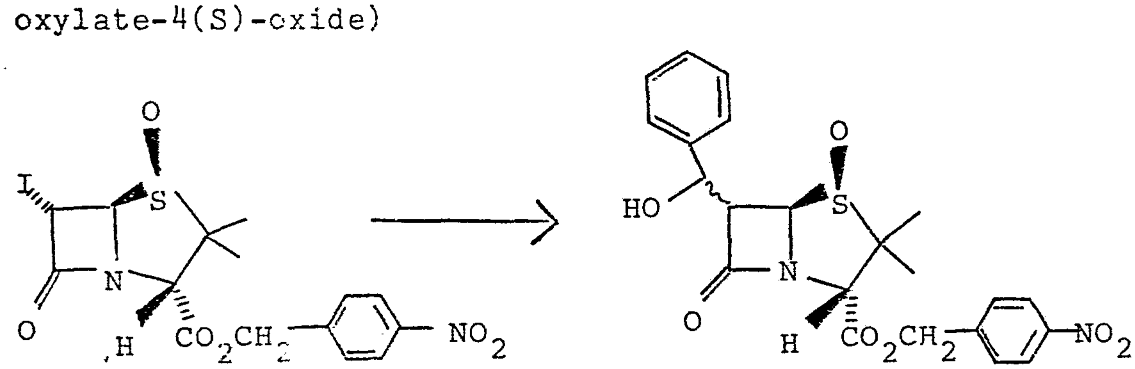

Under an argon atmosphere a stirred solution of 923 mg of 4-nitrobenzyl 6(S)-iodopenicillanate-1(S)-oxide in 20 ml of tetrahydrofuran was cooled to -100°, and treated with 1 ml of a 3M-ethereal solution of methylmagnesium bromide, followed 5 minutes later by 1 ml of benzaldehyde. The solution was warmed to -40° over 30 minutes, was quenched by addition of 1 ml of saturated ammonium chloride and was

BAD ORIGINAL

0018305
HOE 79/F 346

poured into 100 ml of 0.01M-HCl. The pH of the aqueous mixture was readjusted to 2.0 by addition of 2M-HCl, and the mixture was then extracted with ethyl acetate (3 x 25 ml). The combined organic extracts were washed with water (10 ml) and brine (10 ml) and were then dried over sodium sulphate. Evaporation in vacuo yielded an oil which was chromatographed over silica gel. Elution with cyclohexane: ethyl acetate (1:1) yielded one 6(R) alcohol.

δ ($CDCl_3$) 1.07 (3H, s, α-$CH_3$) 1.67 (3H, s, ß-$CH_3$), 3.5 (1H, br, OH), 4.13 (1H, dd $J_{5,6}$ 5Hz, $J_{6,}$ 9Hz, 6-H), 4.53 (1H, d J 5Hz, 5-H), 4.68 (1H, s, 3-H), 5.32 (2H, s, $CH_2$), 5.68 (1H, d J 9Hz, -H), 7.3-8.4 (9H, m, aromatics) and one 6(S) alcohol

δ ($CDCl_3$) 1.13 (3H, s, α-$CH_3$), 1.60 (3H, s, ß-$CH_3$), 3.9 (1H, br, OH), 3.93 (1H, dd $J_{5,6}$ 1Hz, $J_{6,8}$ 5Hz 6-H), 4.55 (1H, s, 3-H), 5.06 (1H d $J_{5,6}$ 1Hz), 5.3 (3H, br s, $CH_2$ and 8-H), 7.3-8.4 (9H, m aromatics) and another 6(S) alcohol

δ ($CDCl_3$) 1.10 (3H, s, α-$CH_3$), 1.63 (3H, s, ß-$CH_3$), 3.3 (1H, br, OH), 4.03 (1H, dd $J_{5,6}$ 1Hz, $J_{6,8}$ 5Hz 6-H), 4.52 (1H, s, 3-H), 4.83 (1H, d $J_{5,6}$ 1Hz), 5.20 (1H, d J 5Hz, 8-H), 5.26 (2H, s, $CH_2$), 7.3-8.4 (9H, m, aromatics).

E X A M P L E 27:

4-Nitrobenzyl 2-/4(R)-(2-benzothiazolyldithio)-2-oxo-3-(phenylhydroxymethyl)-1-azetidinyl7-3-methyl-3-butenate

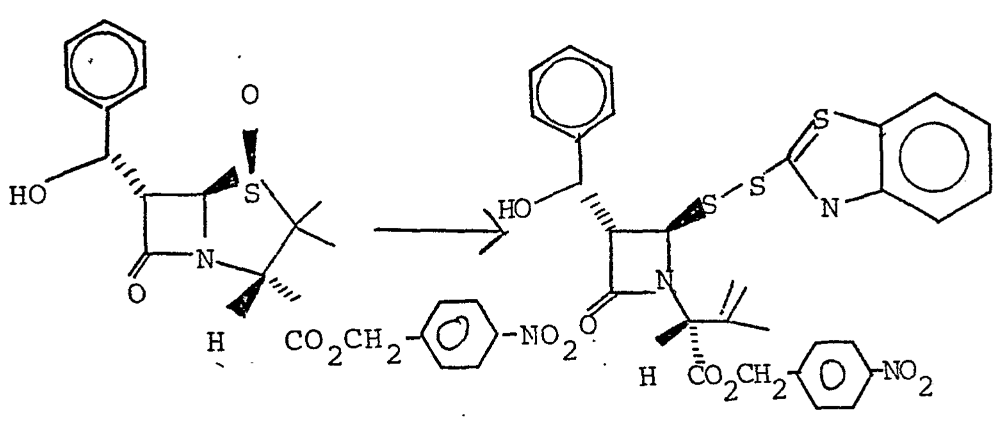

A mixture of 1.32 g of 4-nitrobenzyl 6(S)-(phenylhydroxymethyl)-penicillanate-1-oxide and 485 mg of 2-mercaptobenzothiazole in 100 ml of dry toluene was heated under reflux in an argon atmosphere for 9 hours. The solution was cooled, washed with cold 2M-sodium carbonate (2 x 100 ml) and with brine (100 ml), and was then dried over magnesium sulphate. Evaporation in vacuo and chromatography of the residue over silica gel eluting with hexane: ethyl acetate (3:1 changing to 1:1 and finally 0:1) afforded the above disulphide as a yellow oil (1.67 g, 95 %).
δ ($CDCl_3$) 1.78 (3H, s, $CH_3$), 3.45 (1H, br s, OH), 3.82 (1H, dd J 2Hz, J 5Hz, 3'-H), 4.75-5.13 (6H, m, $CH_2$=C, 4'-H, 2-H, CHO, and $CH_2$), 7.0-8.3 (13H, m, aromatic).

E X A M P L E 28:

4-Nitrobenzyl 2-/4(R)-(2-benzothiazolyldithio)-2-oxo-3-(phenylhydroxymethyl)-1-azetidinyl7-3-hydroxycrotonate

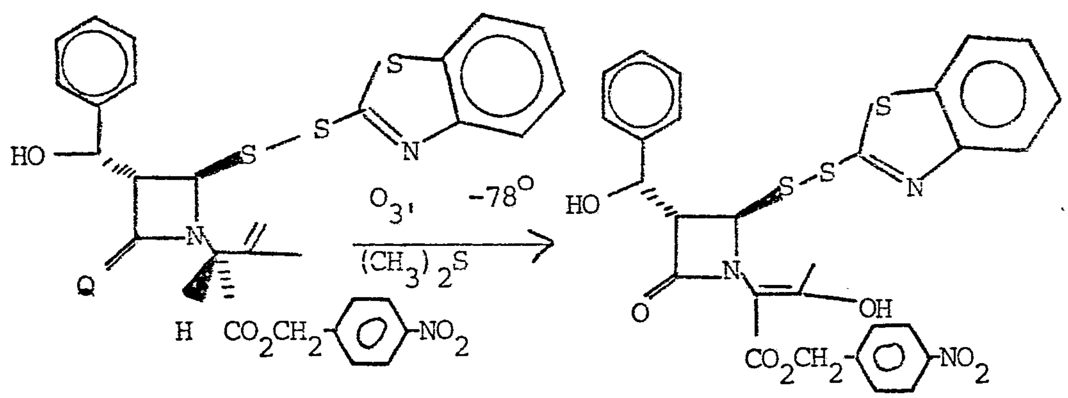


A solution of 1.58 g of 4-nitrobenzyl 2-/4(R)-(2-benzothiazolyldithio)-2-oxo-3(S)-(phenylhydroxymethyl)-1-azetidinyl7-3-methyl-3-butenate in a mixture of 75 ml of dry dichloromethane and 25 ml of methanol was treated at -78° with an oxygen-ozone mixture. 2 ml of dimethyl sulphide was added, and the solution was allowed to warm to room temperature. After evaporation of the solvent in vacuo, the residue was chromatographed on silica gel. Elution with hexane:ethyl acetate (3:1 changing to 2:1) afforded the above enol (861 mg, 54 %).

BAD ORIGINAL

δ $(CDCl_3)$ 1.97 (3H, s, $CH_3$), 3.5-4.0 (2H, m, OH and 3'-H), 4.9-5.2 (4H, m, $CH_2$ and CHO and 4'-H), 7.0-8.3 (13H, m, aromatic), 12.3 (1H, br s, enol)

E X A M P L E 29:

2-Methyl-6(S)-(phenylhydroxymethyl)-3-(4-nitrobenzyloxy-carbonyl)-oxapenem

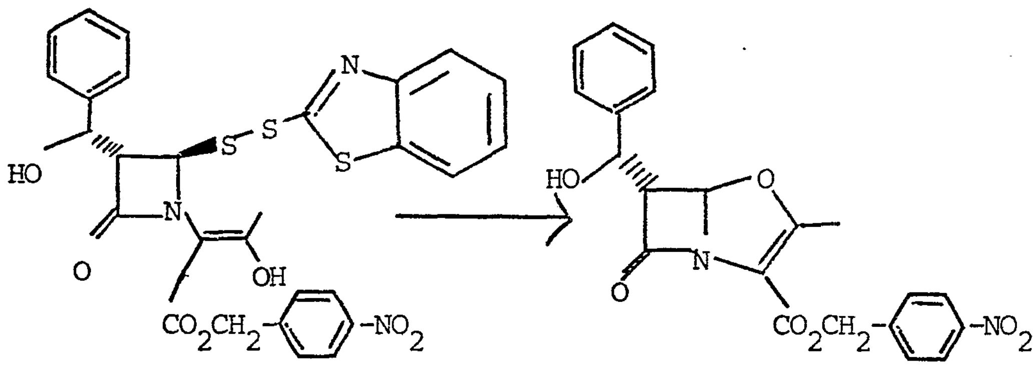

To 52 mg of silver carbonate and 86 mg of triphenylphosphine in 5 ml of dichloromethane was added dropwise a solution of 201 mg of 4-nitrobenzyl 2-/4(R)-(2-benzothia-zolyldithio)-2-oxo-3-(phenylhydroxymethyl)-1-azetidinyl7-3-hydroxycrotonate in 5 ml of dichloromethane. After having been stirred for 10 minutes, the mixture was filtered through Hyflosupercel and evaporated in vacuo to dryness. The residue was chromatographed over silica gel; elution with dichloromethane afforded the above 5(R)-oxapenem ester (98 mg, 72 %).

δ $(CDCl_3)$ 2.28 (3H, s, $CH_3$), 2.8 (1H, br s, OH), 4.0 (1H, dd J 0.5Hz J 5Hz), 5.27 (3H, m, 8-H and $CH_2$), 2.8 (1H, d J 0.5Hz, 5-H), 7.3-8.2 (9H, m, aromatic).

ν max. = 1805 $cm^{-1}$

EXAMPLE 30:

Pivaloyloxymethyl 5(R)-3,3-dimethyl-6(S)-iodo-7-oxo-4-thia-1-azabicyclo/3,2,0/heptane-2(R)-carboxylate

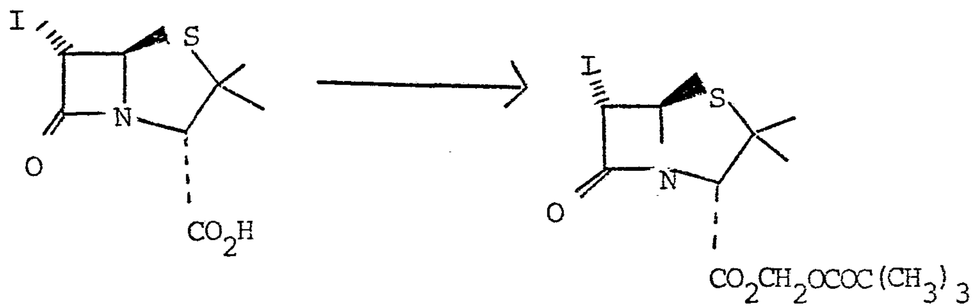

To a solution of 50 g of 5(R)-3,3-dimethyl-6(S)-iodo-7-oxo-4-thia-1-azabicyclo/3,2,0/heptane-2(R)-carboxylic acid in 150 ml of dimethylacetamide was added 21.42 ml of diisopropylamine and a solution of 23.10 ml of pivaloyloxymethyl chloride in 50 ml of dimethylacetamide. 3.05 g of potassium iodide was added after 2 hours, and the mixture was heated for 2 hours at 55°. After filtration the mixture was partitioned between water (1 liter) and ethyl acetate (1 liter). The organic layer was washed with water (3 x 1 liter), 0.01M HCl (until aqueous washes had pH< 3), saturated sodium bicarbonate (1 liter) and brine (1 liter). Evaporation *in vacuo* yielded the ester as a dark brown oil (61.9 g, 92 %) which was pure as judged by TLC (chloroform: diethyl ether:formic acid 7:2:1) and by NMR.

$\nu_{max}$ ($CHCl_3$) 1787, 1760 $cm^{-1}$

$\delta$ ($CDCl_3$) 1.23 (9H, s, $C(CH_3)_3$), 1.50 (3H, s, $\alpha$-$CH_3$), 1.63 (3H, s, $\beta$-$CH_3$), 4.55 (1H, s, 3-H), 5.00 (1H, d J 1.5Hz, 6-H), 5.42 (1H, dJ 0.5Hz, 5-H), 5.75 and 5.85 (2H, d+d J 6Hz, $CH_2$).

EXAMPLE 31:

Pivaloyloxymethyl 5(R)-3,3-dimethyl-6(S)-iodo-7-oxo-4-thia-1-azabicyclo/3,2,0/heptane-2(R)-carboxylate-4(S)-oxide

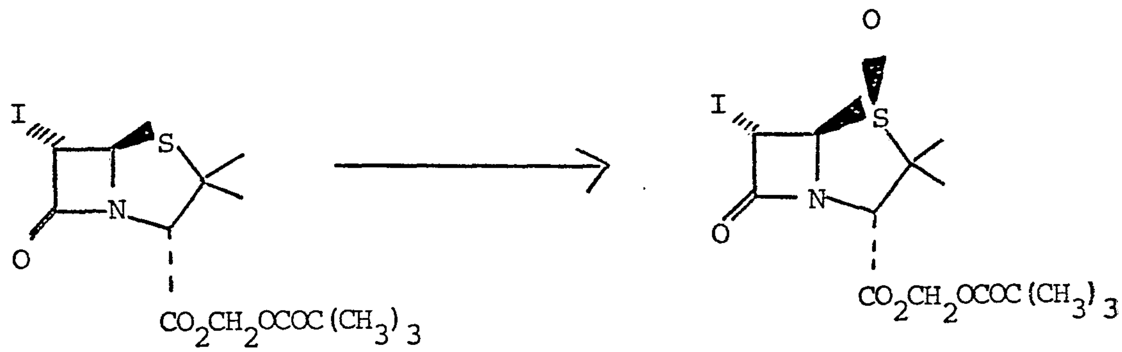

To a solution of 61.9 g of pivaloyloxymethyl 5(R)-3,3-dimethyl-6(S)-iodo-7-oxo-4-thia-1-azabicyclo/3,2,0/-heptane-2(R)-carboxylate in 250 ml of ethyl acetate, cooled to $0^{o}$, was added dropwise the majority of a solution of 31.4 g of m-chloroperoxybenzoic acid in 100 ml of ethyl acetate.

Addition was continued until the yield of sulphoxide, as indicated by TLC (hexane:ethyl acetate, 2:1), was optimised. The crude product was washed with saturated sodium bicarbonate (5 x 500 ml), brine (1 liter), dried over magnesium sulphate and then evaporated to dryness, to yield a dark orange oil. Chromatography over silica gel eluting with hexane:ethyl acetate (2:1) yielded 46.5 g (73 %) of the sulphoxide, as a white foam.

$\nu_{max}$ 1801, 1759 $cm^{-1}$

$\delta$ ($CDCl_3$) 1.25 (12H, s, $\alpha$-$CH_3$ and $C(CH_3)_3$), 1.70 (3H, s, $\beta$-$CH_3$), 4.60 (1H, s, 3-H), 5.16 and 5.30 (2H, d+d J 1Hz, 5-H and 6-H), 5.77 and 6.03 (2H, d+d J 5Hz, $-CH_2-$).

EXAMPLE 32:

Pivaloyloxymethyl 5(R)-3,3-dimethyl-6(S)-(1-hydroxyethyl)-7-oxo-4-thia-1-azabicyclo/3,2,0/heptane-2(R)-carboxylate-4(S)-oxide

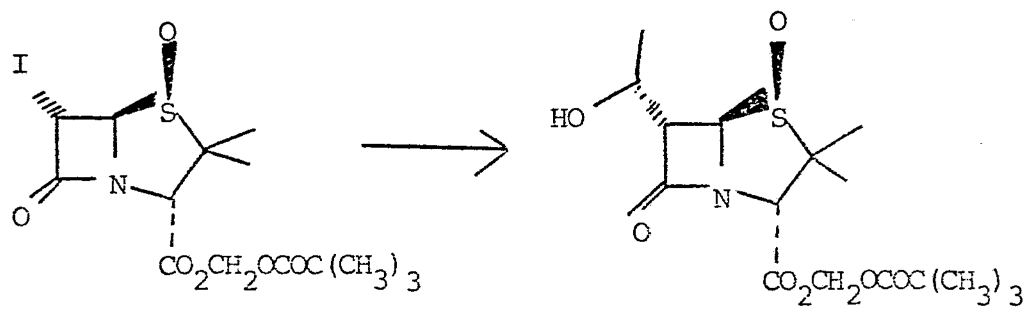

To a stirred solution of 4.43 g of pivaloyloxymethyl 5(R)-3,3-dimethyl-6(S)-iodo-7-oxo-4-thia-1-azabicyclo/3,2,0/-heptane-2(R)-carboxylate-4(S)-oxide in 25 ml of tetrahydrofuran, cooled under an argon atmosphere to -100° was added 5.5 ml of a 3M ethereal solution of methylmagnesium bromide. After having been stirred for 5 minutes, 5 ml of acetaldehyde was added, and the solution was allowed to warm slowly to -40°, and was then quenched with saturated aqueous ammonium chloride. The mixture was evaporated in vacuo to dryness, taken up in 100 ml of ethyl acetate and 20 ml of water. The pH of the aqueous layer was adjusted to 2.0, and the organic layer was further washed with 0.01 M HCl, water and then dried over sodium sulphate, and then evaporated to yield an oil which was chromatographed on silica gel eluting with ethyl acetate:hexane (1:1). Appropriate fractions were combined to yield an oil which was triturated with diethyl ether to yield 804 mg of the above product (22 %).

m.p. 145 - 146°

$\nu_{max}$ ($CHCl_3$) 1790, 1760 (shoulder) $cm^{-1}$

$\delta$ ($CDCl_3$) 1.27 (12H, s, $\alpha$-$CH_3$ and $C(CH_3)_3$), 1.40 (3H, d J 6Hz, $CH_3$), 1.67 (3H, s, $\beta$-$CH_3$), 3.70 (2H, m, OH and 6-H), 4.27 (1H, q J 6Hz, 8-H), 4.50 (1H, s, 3-H), 5.03 (1H, d J 2Hz, 5-H), 5.72 and 6.00 (2H, d+d J 6Hz, $CH_2$).

BAD ORIGINAL

EXAMPLE 33:

Pivaloyloxymethyl 2-/4(R)-(2-benzothiazolyl-dithio)-3(S)-(1-hydroxyethyl)2-oxo-1-azetidinyl7-3-methyl-3-butenate

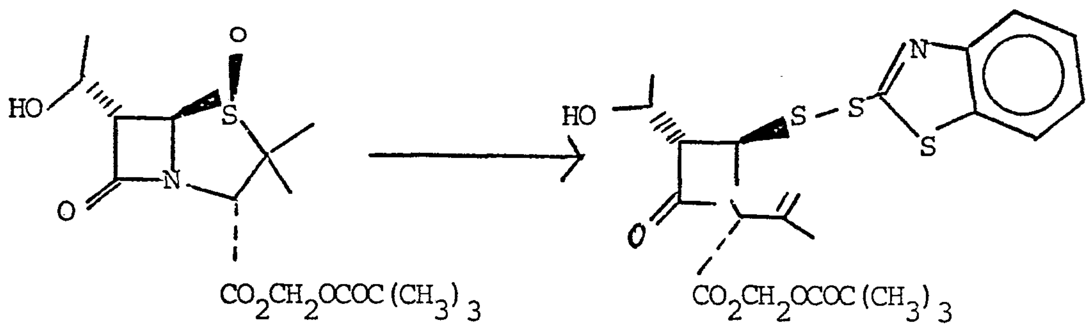

$C_{23}H_{28}N_2O_6S_3$ = 524.66

666 mg of 2-mercaptobenzothiazole and 735 mg of pivaloyloxymethyl 5(R)-3,3-dimethyl-6(S)-(1-hydroxyethyl)-7-oxo-4-thia-1-azabicyclo/3,2,07heptane-2(R)-carboxylate-4(S)-oxide in 15 ml of dry toluene under an argon atmosphere were heated under reflux for 90 min, cooled to $0^o$ and the resulting solid removed by filtration. The filtrate was again cooled to $0^o$, and then extracted with three 25 ml portions of cold saturated $Na_2CO_3$. After being further washed with water (3 x 25 ml), and brine (2 x 10 ml), the organic solution was dried over sodium sulphate, and evaporated to dryness. The crude product was chromatographed over silica gel eluting with ethyl acetate:hexane (1:2) to yield 574 mg of the above product in purified form (56 %).

$\gamma_{max}$ ($CHCl_3$) 1759 $cm^{-1}$

$\delta$ ($CDCl_3$) 1.23 (9H, s, $(CH_3)_3$), 1.37 (3H, d J 6Hz, $CH_3$), 1.97 (3H, bs, $CH_3$), 2.67 (1H, bs, OH), 3.55 (1H, dd J 2Hz, 5Hz, 3-H), 4.20 (1H, dq J 5Hz, 6Hz, CHOH), 4.87 (1H, bs), 5.12 (1H, bs), 5.20 (1H, m), 5.29 (1H, d, J 2Hz, 4-H), 5.72, 5.88 (2H, $J_{A,B}$ 5Hz, $CH_2$), 7.3-8.10 (4H, m, $C_6H_4$).

E X A M P L E 34:

Pivaloyloxymethyl 2-[4(R)-(2-benzothiazolyl-dithio)-3(S)-(1-hydroxyethyl)2-oxo-1-azetidinyl]-3-hydroxycrotonate

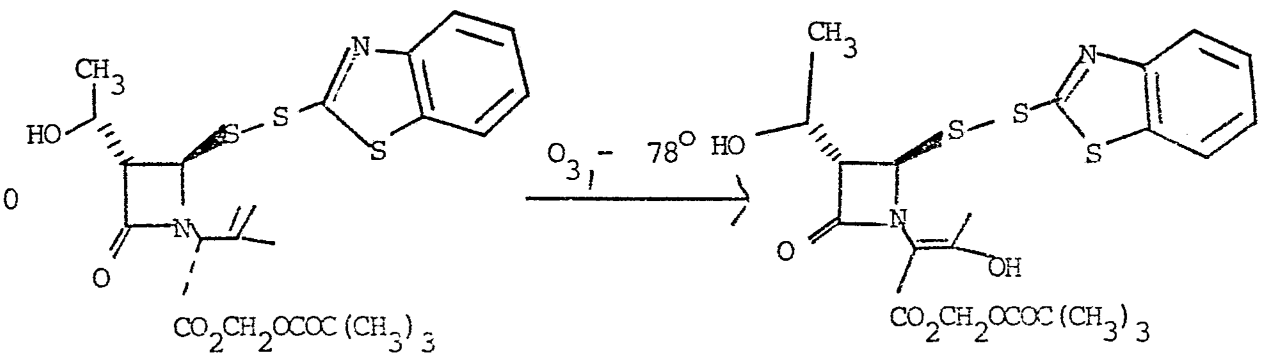


574 mg of Pivaloyloxymethyl 2-[4(R)-(2-benzothiazolyldithio)-3(S)-(1-hydroxyethyl)-2-oxo-1-azetidinyl]-3-methyl-3-butenate in a mixture of 25 ml of dry dichloromethane and 8 ml of methanol was cooled to -78° and treated with an oxygen-ozone mixture until the solution went blue. After addition of 1.0 ml of dimethyl sulphide the solution was allowed to warm to room temperature, and was then evaporated *in vacuo*. The residue was twice chromatographed, firstly over silica gel eluting with ethyl acetate:hexane (1:2), and secondly over silica gel eluting ethyl acetate: dichloromethane (1:2), to yield 330 mg of the above product as a white foam (57 %).

$_{max}$ ($CHCl_3$) 1764 $cm^{-1}$

$\delta$ ($CDCl_3$) 1.18 (9H, s, $(CH_3)_3$), 1.40 (3H, d J 6Hz, $CH_3$), 2.17 (3H, s, $CH_3$), 2.5 (1H, bs, OH), 3.50 (1H, dd J 5Hz, 3-H), 4.23 (1H, dq J 5Hz, 6Hz, CHOH), 5.15 (1H, d J 2Hz, 4-H), 5.83 (2H, s, $CH_2$), 7.3-8.0 (4H, m, $C_6H_4$), 12.17 (1H, bs, OH).

E X A M P L E 35:

2-Methyl-6(R)-(1-hydroxyethyl)-3-pivaloyloxymethyloxy-carbonyl)-oxapenem

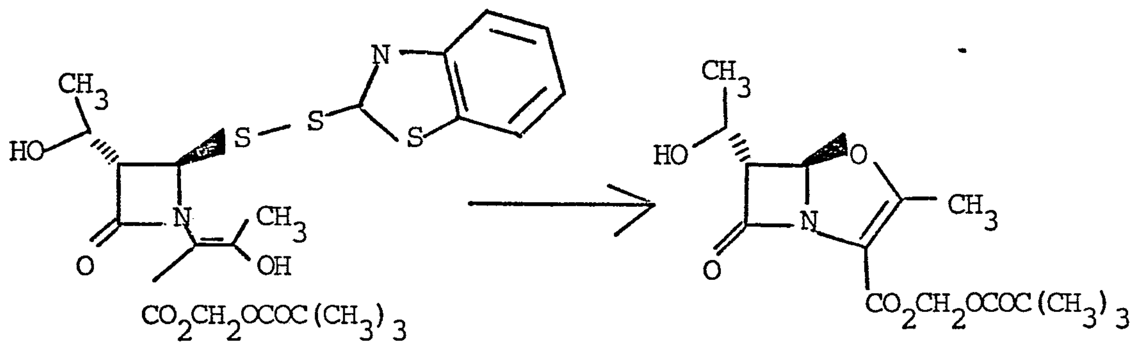

To 67 mg of silver carbonate and 86 mg of triphenylphosphine in 3 ml of dichloromethane was added dropwise a solution of 170 mg of pivaloyloxymethyl-2-/4(R)-(2-benzothiazolyldithio)-2-oxo-3-(1-hydroxyethyl)-1-azetidinyl7-3-hydroxycrotonate in 3 ml of dichloromethane. After being stirred for 10 minutes, the mixture was filtered through Hyflosupercel; the filtrate was evaporated to dryness (250 mg, yellow oil), and the residue was rapidly chromatographed over silica gel. Elution with dichloromethane followed by dichloromethane:ethyl acetate (2:1) afforded the oxapenem ester (31 mg, 29 %) as a yellow oil.

$\nu$ max ($CDCl_3$) 1807, 1754 cm-1

$\delta$ ($CDCl_3$) 1.22 (9H, s, $C(CH_3)_3$), 1.40 (3H, d J 6Hz, $CH_3$), 2.28 (3H, s, $CH_3$), 3.73 (1H, dd J$<$1Hz J 5Hz, 6-H), 4.23 (1H, m, 8-H), 5.2 (1H, broad, OH), 5.87 (3H, m, 5-H and $CH_2$).

EXAMPLE 36:

Pivaloyloxymethyl 5(R)-3,3-dimethyl-6(S)-(2-hydroxy-2-propyl)-7-oxo-4-thia-1-azabicyclo/3,2,0/heptane-2(R)-carboxylate-4(S)-oxide

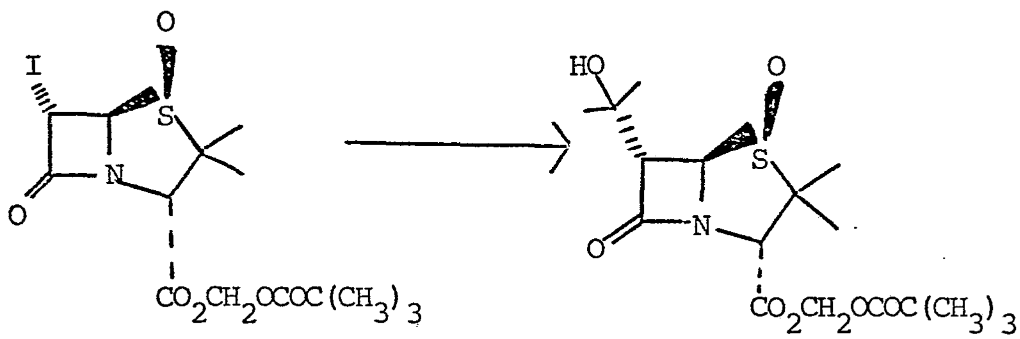

To a solution of 2.83 g of dried pivaloyloxymethyl 5(R)-3,3-dimethyl-6(S)-iodo-7-oxo-4-thia-1-azabicyclo/3,2,0/heptane-2(R)-carboxylate-4(S)-oxide, in 50 ml of tetrahydrofuran cooled under argon to -100° was added 3.6 ml of a 3M ethereal solution of methylmagnesium bromide. After the mixture had been stirred for 5 minutes, 4.6 ml of acetone was added. The mixture was allowed to warm to -40° over 30 minutes, was quenched by addition of saturated ammonium chloride, was warmed to room temperature, and then poured into 0.01M HCl. The pH was readjusted to 2.0 with 2 M HCl, and then the mixture was extracted with ethyl acetate (3 x 100 ml). The combined organic extracts were backwashed with water (2 x 100 ml) and brine, and were dried over anhydrous sodium sulphate. Evaporation *in vacuo* yielded a colourless oil, which on trituration with ether yielded the alcohol as colourless crystals (2.12 g, 87 %), m.p. 119 - 20°.

$\nu_{max}$ ($CHCl_3$) 1790, 1759 $cm^{-1}$

$\delta$ ($CDCl_3$) 1.25 (9H, s, $C(CH_3)_3$), 1.30 (3H, s, $\alpha$-$CH_3$), 1.38 (3H, s, $CH_3$), 1.50 (3H, s, $CH_3$), 1.70 (3H, s, ß-$CH_3$), 2.97 (1H, bs, OH), 3.63 (3H, d J 2Hz, 6-H), 4.33 (1H, s, 3-H), 5.00 (1H, d J 2Hz, 3-H), 5.72 and 5.97 (2H, d+d J 5Hz, $CH_2$).

E X A M P L E 37:

Pivaloyloxymethyl 2-/4(R)-(2-benzothiazolyldithio)-3(S)-(2-hydroxy-2-propyl)-2-oxo-1-azetidinyl/-3-methyl-3-butenate

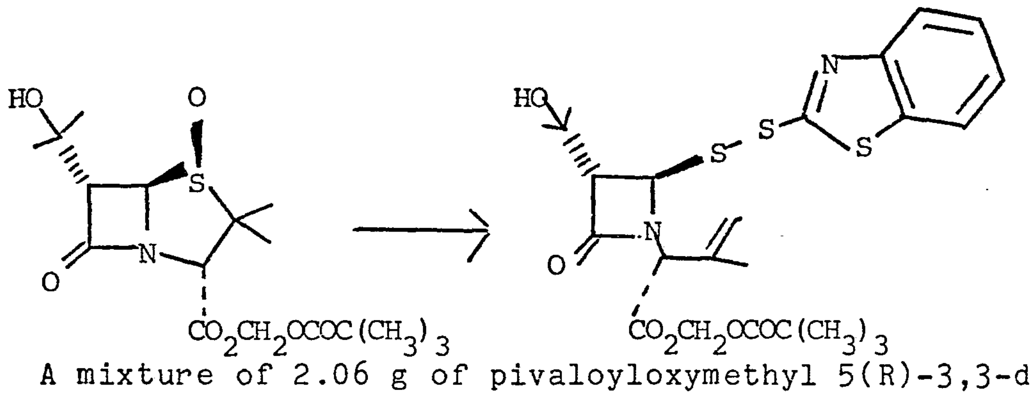

A mixture of 2.06 g of pivaloyloxymethyl 5(R)-3,3-dimethyl-6(S)-(2-hydroxy-2-propyl)-7-oxo-4-thia-1-azabicyclo/3,2,0/heptane-2(R)-carboxylate-4(S)-oxide and 880 mg of 2-mercaptobenzothiazole in 50 ml of dry toluene was heated under reflux under nitrogen for 8 hours. Evaporation of the solvent in vacuo, and chromatography of the residue over silica gel eluting with cyclohexane:ethyl acetate (2:1) afforded pure disulphide (1.16 g, 41 %) as a yellow sirup.

$\nu_{max}$ ($CHCl_3$) 1735, 1760 (shoulder) $cm^{-1}$

$\delta$ ($CDCl_3$) 1.20 (9H, s, $C(CH_3)_3$), 1.37 (6H, s, $C(CH_3)_2$), 1.92 (3H, s, $CH_3$-C=), 2.93 (1H, bs, OH), 3.48 (1H, d J 2.5Hz, 3-H), 4.82 (1H, bs, 2-H), 5.1-5.2 (2H, m, $CH_2$=), 5.3 1H, d J 2.5Hz, 4'-H), 5.65 and 5.82 (2H, d+d J 6Hz, $CH_2$), 7.3-8.0 (4H, m, $C_6H_4$).

E X A M P L E 38:

Pivaloyloxymethyl 2-/4(R)-(2-benzothiazolyldithio)-3(S)-(2-hydroxy-2-propyl)-2-oxo-1-azetidinyl/-3-hydroxycrotonate

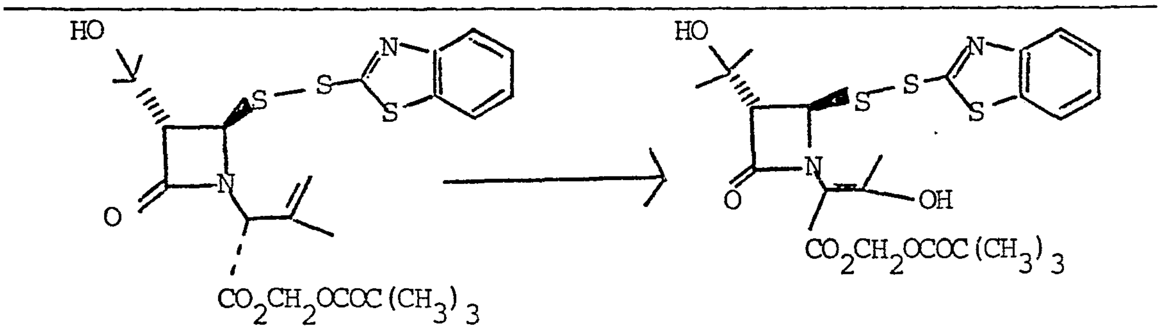

A solution of 1.15 g of pivaloyloxymethyl 2-/4(R)-(2-benzothiazolyldithio)-3(S)-(2-hydroxy-2-propyl)-2-oxo-1-azetidinyl7-3-methyl-3-butenate in a mixture of 20 ml of dry dichloromethane and 5 ml of methanol was treated at -78° with an oxygen-ozone mixture until a blue colouration was observed. 1.5 ml of dimethyl sulphide was added, and the solution allowed to warm to room temperature. The solution was evaporated *in vacuo*, and the residue chromatographed on silica gel eluting with hexane:ethyl acetate (3:1) to give the above crotonate.

$\nu_{max}$ ($CHCl_3$) 1769 $cm^{-1}$

$\delta$ ($CDCl_3$) 1.20 (9H, bs, $C(CH_3)_3$), 1.36 and 1.40 (6H, s+s $(CH_3)_2C$), 2.13 (3H, s, $CH_3$), 3.30 (1H, d J5,6 2Hz, 6-H), 3.3-4.0 (1H, broad, OH), 5.22 (1H, d $J_{5,6}$ 2Hz, 5-H), 5.77 (2H, bs, $CH_2$), 7.3-8.0 (4H, m, $C_6H_4$), 13.20 (1H, bs, C=C-OH).

EXAMPLE 39:

2-Methyl-6(R)-(2-hydroxy-2-propyl)-3-pivaloyloxymethyloxycarbonyl)-oxapenem

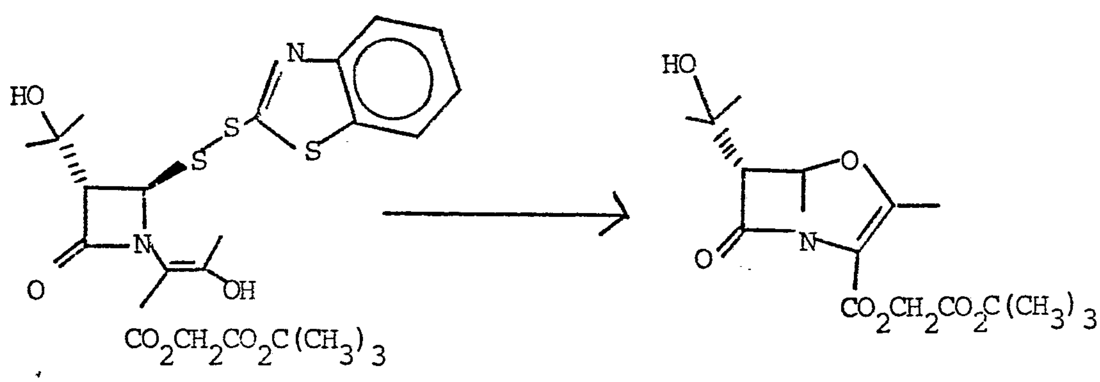

To 44 mg of silver carbonate and 74 mg of triphenylphosphine in 4 ml of dichloromethane was added dropwise a solution of 155 mg of pivaloyloxymethyl 2/4(R)-(2-benzothiazolyldithio)-3(S)-(2-hydroxy-2-propyl)-2-oxo-1-azetidinyl7-3-hydroxycrotonate, in 3 ml of dichloromethane. After being stirred for 10 minutes, the mixture was filtered through Hyflosupercel and evaporated *in vacuo* to dryness. The residue was chromatographed over silica gel elution with dichloro-

BAD ORIGINAL

methane followed by dichloromethane:ethyl acetate (2:1) afforded predominantly the 5(R)-oxapenem ester 22 mg, 23 %) as an oil.

ν max 1805 cm-1

δ ($CDCl_3$) 1.22 (9H, bs, $C(CH_3)_3$), 1.38 and 1.41 (6H, s+s $(CH_3)_2C$), 2.88 (3H, s, $CH_3$), 3.62 (1H, d $J_{5,6}$ 2Hz, 6-H), 5.83 (3H, m, $CH_2$ and 5-H).

EXAMPLE 40:

Pivaloyloxymethyl 5(R)-3,3-dimethyl-6(S)-(phenylhydroxymethyl)-7-oxo-4-thia-1-azabicyclo/3,2,0/heptane-2(R)-carboxylate-4(S)-oxide

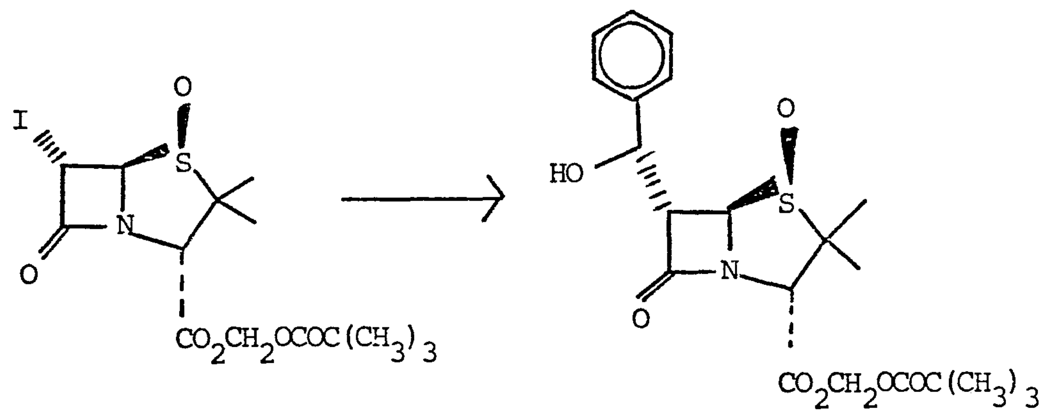

To a solution of 3.13 g of pivaloyloxymethyl 5(R)-3,3-dimethyl-6(S)-iodo-7-oxo-4-thia-1-azabicyclo/3,2,0/heptane-2(R)-carboxylate-4(S)-oxide in 60 ml of tetrahydrofuran cooled under argon to -100° was added 4 ml of a 3M ethereal solution of methylmagnesium bromide. After the mixture had been stirred for 5 minutes 6.9 ml of benzaldehyde was added. The mixture was allowed to warm to -40°, was quenched by the addition of 15 ml of saturated ammonium chloride, was warmed to room temperature and was then poured into 0.01M HCl. The pH was readjusted to 2.0 with 2M HCl, and then the mixture was extracted with ethyl acetate (3 x 100 ml). The combined organic extracts were backwashed with water (2 x 100 ml), and brine, and were dried over anhydrous sodium sulphate. Evaporation *in vacuo* yielded a yellow oil (3.0 g). Trituration with hexane, precipitated 122 mg of the above product, chromatography of the residue

over silica gel eluting with ethyl acetate:hexane (1:2) afforded further 6(S)-alcohol (717 mg, 24 %), together with 1.33 g of mixed fractions.

$\nu_{max}$ ($CHCl_3$) 1792, 1759 $cm^{-1}$

$\delta$ ($CDCl_3$) 1.22 (12H, s, $C(CH_3)3$ and $\alpha$ -$CH_3$), 1.65 (3H, s, $\beta$-$CH_3$), 2.87 (1H, d J 4Hz, OH), 3,96 (1H, dd $J_{5,6}$ 2Hz $J_{6,8}$ 6Hz, 6-H), 4.43 (1H, s, 3-H), 4.77 (1H, d $J_{5,6}$ 2Hz), 5.1 (1H, m, 8-H), 5.62 and 5.87 (2H, d+d J 6Hz, $CH_2$), 7.33 (5H, bs, $C_6H_5$).

E X A M P L E 41:

Pivaloylmethyl 2-/4(R)-(2-benzothiazolyldithio)-3(S)-(phenylhydroxymethyl)-2-oxo-1-azetidinyl7-3-methyl-3-butenate

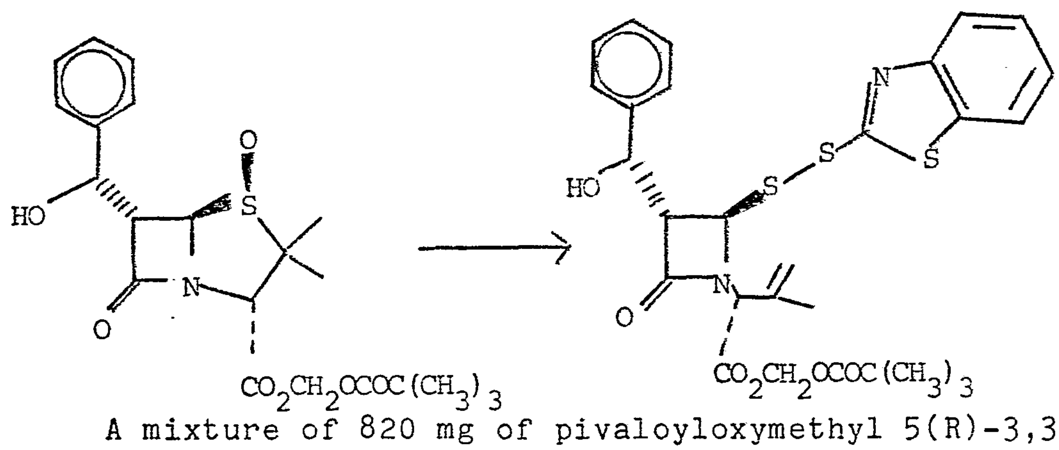

A mixture of 820 mg of pivaloyloxymethyl 5(R)-3,3-dimethyl-6(S)-(phenylhydroxymethyl)-7-oxo-4-thia-1-azabicyclo/3,2,07heptane-2(R)-carboxylate-4(S)-oxide and 313 mg of 2-mercaptobenzothiazole in 25 ml of toluene was heated in an argon atmosphere under reflux for 4 hours. The solvent was evaporated *in vacuo* to afford the above disulphide (999 mg, 91 %).

$\delta$ ($CDCl_3$) 1.18 (9H, s, $C(CH_3)_3$), 1.80 (3H, bs, $C=C-CH_3$), 3.43 (1H, bs, OH), 3.82 (1H, dd $J_{3,4}$ 2Hz, $J_{3,5}$ 6Hz, 3-H), 4.78 (1H, s, 2-H), 4.9-5.2 (4H, m, 4'-H, 5-H, $C=CH_2$), 5.63 and 5.80 (2H, d+d $J_{AB}$ 6Hz, $CH_2$), 7.0-8.0 (9H, m, $C_6H_5$ and $C_6H_4$).

EXAMPLE 42:

Pivaloyloxymethyl 2-/4(R)-(2-benzothiazolyldithio)-3(S)-(phenylhydroxymethyl)-2-oxo-1-azetidinyl7-3-hydroxy-crotonate

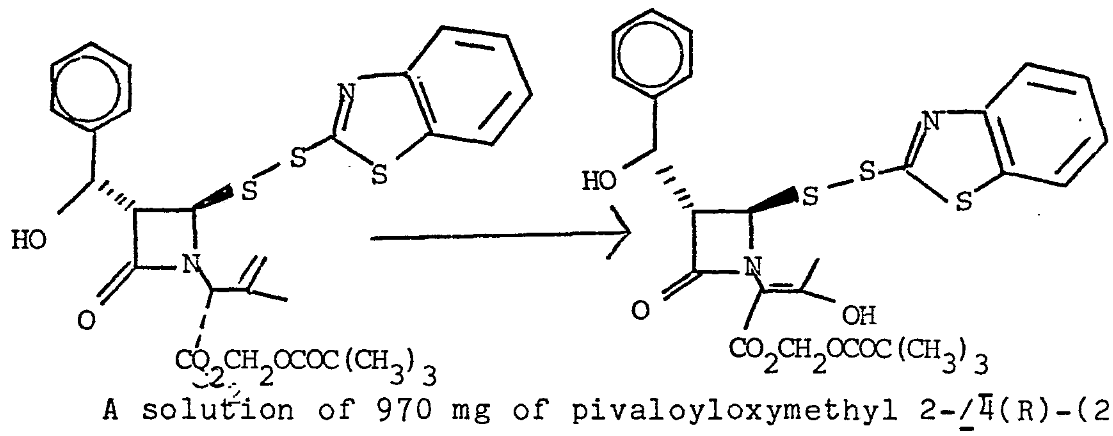

A solution of 970 mg of pivaloyloxymethyl 2-/4(R)-(2-benzothiazolyldithio)-3(S)-phenylhydroxymethyl)-2-oxo-1-azetidinyl7-3-methyl-3-butenate in a mixture of 15 ml of dichloromethane and 5 ml of methanol was treated at -78° with an oxygen-ozone mixture until a blue colouration was observed. 1.25 ml of dimethyl sulphide was added and the solution allowed to warm to room temperature. The solution was evaporated in vacuo and the residue chromatographed on silica gel eluting with hexane:ethyl acetate = 2:1 to give the above crotonate (398 mg, 42 %) as a white foam.

max ($CHCl_3$) 1768 $cm^{-1}$

($CDCl_3$) 1.17 (9H, s, $C(CH_3)_3$), 1.97 (3H, bs, $C=C-CH_3$), 3-4 (1H, broad, OH), 3.73 (1H, dd $J_{3,4}$ 2Hz, $J_{3,5}$ 6Hz, 3-H), 4.95-5.13 (2H, m, 3-H and 5-H), 5.67 (2H, bs, $CH_2$), 7.0-8.0 (9H, m, $C_6H_5$ and $C_6H_4$), 12.17 (1H, bs, OH).

0018305
HOE 79/F 346

EXAMPLE 43:

2-Methyl-6(R)-(phenylhydroxymethyl)-3-pivaloyloxymethyloxy-carbonyl)-oxapenem

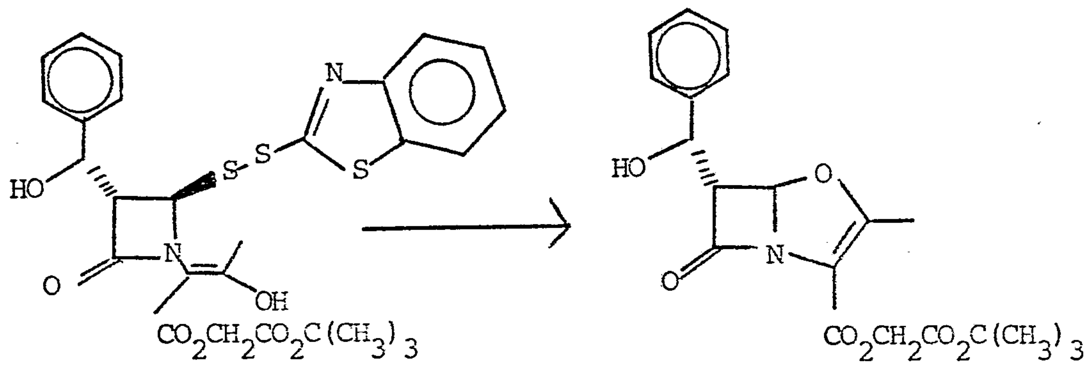

To 35 mg of silver carbonate and 57 mg of triphenyl phosphine in 4 ml of dichloromethane was added dropwise a solution of 130 mg of pivaloyloxymethyl 2-/4(R)-(2-benzothiazolyldithio)-3(S)-(phenylhydroxymethyl)-2-oxo-1-azetidinyl7-3-hydroxy-crotonate in 2 ml of dichloromethane. After being stirred for 10 minutes the mixture was filtered through Hyflosupercel and evaporated in vacuo to dryness. The residue was rapidly chromatographed over silica gel, elution with dichloromethane followed by dichloromethane: ethyl acetate (2:1) afforded the predominantly 5(R)-oxapenem ester (21 mg, 25 %) as an oil.

$\nu_{max}$ 1730, 1750, 1808 $cm^{-1}$

$\delta$ ($CDCl_3$) 1.12 (9H, s, $C(CH_3)_3$), 2.15 (3H, s, $CH_3$), 3.88 (1H, dd $J_{6,5}$ 0.5Hz, $J_{6,8}$ 5Hz, 6-H), 5.04 (1H, d $J_{8,6}$ 5Hz, 8-H), 5.63 (1H, d $J_{5,6}$ 0.5Hz, 5-H), 5.72 (2H, s, $CH_2$), 7.26 (5H, s, $C_6H_5$)

Patent Claims:

1. A process for the production of a compound of the general formula I

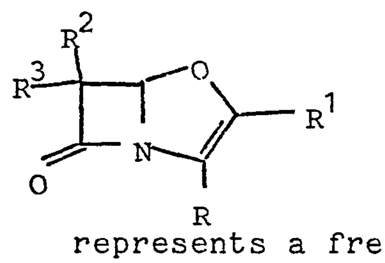

(I)

in which R represents a free or esterified carboxyl group,

$R^1$ represents a hydrogen atom or a straight or branched chain, unsubstituted or substituted aliphatic group, and

$R^2$ and $R^3$, which may be the same or different, each represents a hydrogen or halogen atom, an amine or substituted amine group, or an unsubstituted or substituted straight or branched chain aliphatic group,

or a salt thereof, which comprises reacting a compound of the general formula II

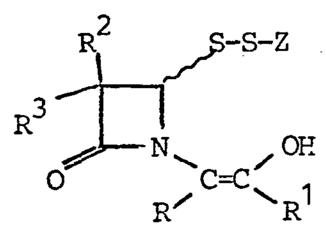

(II)

in which R, $R^1$, $R^2$ and $R^3$ are as defined above, and

Z represents an unsubstituted or substituted aromatic heterocyclic radical with up to 15 carbon atoms and at least one ring nitrogen atom and optionally a further ring heteroatom, which radical is bonded to the dithio group by one of its ring carbon atoms, which is bonded to a ring nitrogen atom by a double bond, or

Z represents an acyl radical derived from an organic carboxylic acid or thiocarboxylic acid,

with a tervalent organophosphorus compound and, if desired, simultaneously or subsequently with a thiophilic agent as hereinbefore defined and, if desired, carrying out any one or more of the following steps in any appropriate order:

(i) converting an ester into another ester or the free acid,

(ii) converting a free acid into an ester or a salt,

(iii) converting a salt into the free acid or another salt.

2. A process as claimed in claim 1, wherein the compound of the general formula II is treated with a tervalent organophosphorus compound, and a resulting monosulphide of the general formula IIa

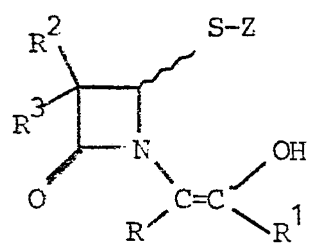

(IIa)

in which R, $R^1$, $R^2$, $R^3$ and Z are defined as in claim 1, is treated with a thiophilic agent and, if desired, any one or more of the steps (i) to (iii) defined in claim 1 are carried out.

3. A process for the production of a compound of the general formula I as defined in claim 1, wherein a compound of the general formula IIa

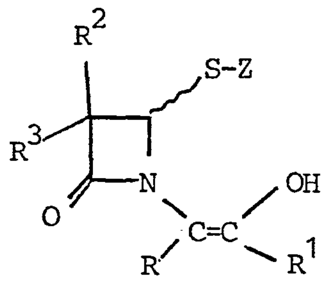

(IIa)

in which R, $R^1$, $R^2$, $R^3$ and Z are defined as in claim 1, is treated with a thiophilic agent and, if desired, any one or more of the steps (i) to (iii) defined in claim 1 are carried out.

4. A process as claimed in claim 1 or claim 2, wherein the compound of formula II is produced by converting a compound of formula III

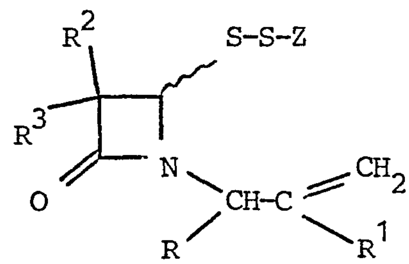

(III)

in which R, $R^1$, $R^2$, $R^3$ and Z are as defined in claim 1, into the corresponding enol.

5. A process as claimed in claim 3, wherein the compound of formula IIa is produced by converting a compound of formula IIIa

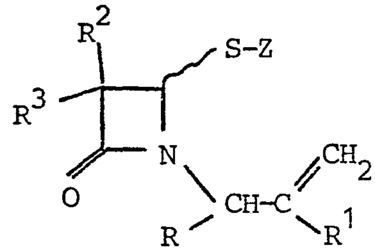

(IIIa)

in which R, $R^1$, $R^2$, $R^3$ and Z are as defined in claim 1, into the corresponding enol.

6. A process as claimed in claim 5, wherein the compound of formula IIIa is produced by desulphurizing the corresponding compound of formula III by treatment with a tervalent organophosphorus compound.

7. A process as claimed in claim 4 or claim 6, wherein a compound of formula III is produced by reacting a compound of formula IVc

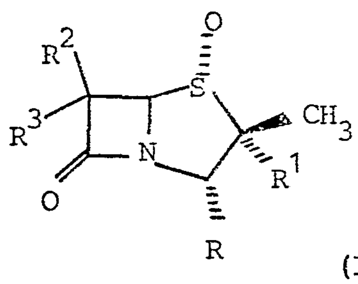
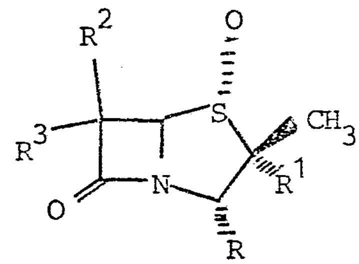

(IVc)

in which R, $R^2$ and $R^3$ are as defined in claim 1, with a compound of formula V

$$H - S - Z \qquad (V)$$

in which Z is as defined in claim 1.

8. A process as claim in claim 7, wherein there is introduced into a compound of formula IVc in which $R^2$ and $R^3$ are the same and both represent hydrogen atoms or one represents a hydrogen atom and the other a halogen atom, one or two amine or aliphatic radicals $R^2$ and/or $R^3$ as defined in claim 1.

9. A process as claimed in claim 8, wherein there is introduced an unsubstituted or substituted straight or branched chain aliphatic group.

10. A process as claimed in claim 9, wherein there is introduced a lower aliphatic group substituted by a hydroxy that may itself be substituted.

BAD ORIGINAL

11. A process as claimed in claim 10, wherein the group introduced is a hydroxymethyl, phenylhydroxymethyl, 1-hydroxyethyl or 2-hydroxy-2-propyl group.

12. A process as claimed in any one of claims 8 to 11, wherein in the starting material of formula IVc one of $R^2$ and $R^3$ represents a hydrogen atom and the other represents an iodine atom.

13. A process as claimed in any one of claims 8 to 12, wherein the compound of formula IVc is treated with an organometallic reagent and then with an aldehyde or ketone.

14. A process as claimed in claim 7, wherein a compound of formula IVc in which $R^1$ represents a methyl group is produced by oxidising a compound of formula VI

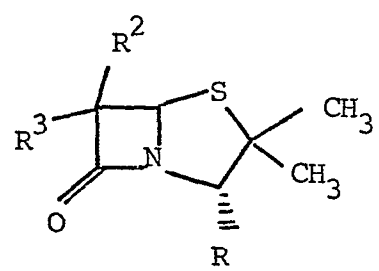

(VI)

in which R, $R^2$ and $R^3$ are as defined in claim 1.

15. A process as claimed in claim 14, wherein the compound of formula VI is prepared from a compound of the general formula VII

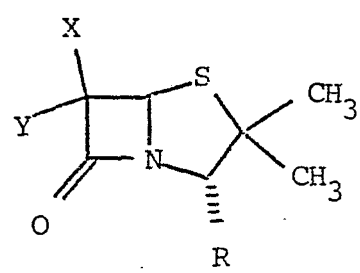

(VII)

in which X and Y are the same and each represents a halogen atom, or X represents a hydrogen atom and Y represents a halogen atom, and R is as defined in claim 1, by catalytic hydrogenation to give a compound of formula VII in which $R^2$ and $R^3$ both represent hydrogen atoms, or by introducing one or two radicals $R^2$ and $R^3$.

16. A process as claimed in claim 15, wherein the compound of formula VII is produced from 6-amino-penicillanic acid by treatment with nitrous acid and a halogenating agent.

17. A process as claimed in claim 1, claim 2 or claim 5, wherein the tervalent organophosphorus compound has the general formula

$$PR^4R^5R^6$$

wherein $R^4$, $R^5$ and $R^6$, which may be the same or different, each represents a straight or branched chain unsubstituted or substituted hydrocarbon group, or a straight or branched chain unsubstituted or substituted hydrocarbon group in which one or more carbon atoms are replaced by hetero atoms.

18. A process as claimed in claim 17, wherein the tervalent organophosphorus compound is triphenylphosphine, tributylphosphine, trimethylphosphite and triethylphosphite.

19. A process as claimed in claim 17, wherein in $PR^4R^5R^6$ one or more of the groups $R^4$, $R^5$ and $R^6$ comprises an insoluble polymer.

20. A process as claimed in claim 17, wherein in $PR^4R^5R^6$ one or more of the groups $R^4$, $R^5$ and $R^6$ comprises a

cationic or anionic centre.

21. A process as claimed in any one of claims 1 to 3, wherein the thiophilic agent is a thiophilic silver, copper, zinc, nickel, iron, cadmium, mercury or cobalt salt.

22. A process as claimed in claim 21, wherein the thiophilic agent is $Ag_2CO_3$, AgO, $Cu(acac)_2$, $Cu(CO_3)_2$, $Cu_2CO_3$, $CuOR^7$ in which $R^7$ represents an alkyl group or cycloalkyl group.

23. A process as claimed in claim 21, wherein the thiophilic agent is

(i) $AgNO_3$, $AgOSO_2CF_3$, $AgOSO_2Me$, $AgBF_4^{\ominus}$, $AgPF_6^{\ominus}$, CuCl, CuBr, $CuCl_2$, $CuBr_2$, $CuOSO_2Me$, $CuOSO_2CF_3$, $CuSO_4$, $Cu(NO_3)_2$ or the corresponding nickel, zinc, iron or cobalt salt, and

(ii) a base.

24. A process as claimed in claim 1, wherein Z represents a monocyclic five membered thiadiazacyclic, thiatriazacyclic, oxadiazacyclic, oxatriazacyclic, diazacyclic, oxazacyclic or thiazacyclic radical of aromatic character, or a benzdiazacyclic, benzoxacyclic or benzthiacyclic radical, wherein the heterocyclic moiety is five membered and of aromatic character, a substitutable ring nitrogen atom being optionally substituted.

25. A process as claimed in claim 1, wherein Z represents 1-methyl-imidazol-2-yl, 1,3-thiazol-2-yl, 1,3,4-thiadiazol-2-yl, 1,3,4,5-thiatriazol-2-yl, 1,3-oxazol-2-yl, 1,3,4-oxadiazol-2-yl, 1,3,4,5-oxatriazol-2-yl, 2-quinolyl, 1-methyl-benzimidazol-2-yl, benzoxazol-2-yl or benzthiazol-2-yl group.

26. A process as claimed in claim 1, wherein Z represents

an acyl radical derived from an unsubstituted or substituted aliphatic, cycloaliphatic, araliphatic or aromatic acyl or thioacyl group having up to 18 carbon atoms.

27. A process as claimed in claim 1, wherein Z represents the group

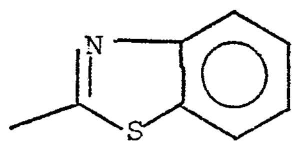 or 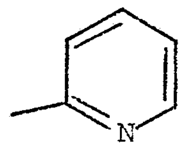 .

28. A process as claimed in claim 1, carried out substantially as described in any one of Examples 11, 13 to 16, 25, 29, 35, 39 and 43.

29. A process as claimed in claim 2 or 3, carried out substantially as described in any one of Examples 9, 10, 12, 17 and 18.

30. A compound of the general formula I as defined in claim 1, whenever produced by a process as claimed in any one of claims 1 to 29.

31. A compound as claimed in claim 30, wherein $R^1$ represents an unsubstituted or substituted alkyl, alkenyl, or alkynyl group having up to 8 carbon atoms.

32. A compound as claimed in claim 31, wherein $R^1$ represents an unsubstituted or substituted alkyl, alkenyl, or alkynyl group having up to 4 carbon atoms.

33. A compound as claimed in claim 32, wherein $R^1$ represents an unsubstituted or substituted methyl group.

34. A compound as claimed in any one of claims 2 to 33, wherein an aliphatic group $R^1$ is substituted by one or

more substituents selected from halogen atoms; oxo groups; hydroxyl and mercapto groups, alkoxy and alkylthio groups; alkylcarbonyl groups, carboxy, alkoxycarbonyl and alkylthiocarbonyl groups: alkanoyloxy and alkanoylthio groups; carbamoyl and carbomoyloxy groups and carbamoyl and carbamoyloxy groups substituted on the nitrogen atom by one or two groups selected from alkyl and aryl groups and the corresponding unsubstituted and substituted groups in which the oxygen atom or each or either oxygen atom is replaced by a sulphur atom; nitro, cyano and azido groups; amido and imido groups; imino, amino, mono- and di-alkylamino, mono- and di-arylamino groups and N,N-alkylarylamino groups; acylamino groups; sulphinyl, sulphonyl and sulphonamido groups; cycloalkyl groups, aryl, aryloxy, arylthio, aryloxycarbonyl, arylthiocarbonyl, arylcarbonyloxy, arylcarbonylthio, aralkoxycarbonyl, aralkylthiocarbonyl, aralkylcarbonyloxy, aralkylcarbonylthio, aralkoxy and aralkylthio groups; aromatic and non-aromatic heterocyclic groups, aromatic and non-aromatic heterocyclicoxy groups and aromatic and non-aromatic heterocyclicthio groups.

35. A compound as claimed in claim 34, wherein $R^1$ represents a methyl group substituted by one or more halogen atoms, an esterified or etherified methyl group, a methyl group substituted by a -SHet group in which Het represents an aromatic or non-aromatic heterocyclic group, a methyl group substituted by an amino, mono- or di-lower alkylamino group, mono- or di-arylamino group or lower alkyl arylamino group, or a methyl group substituted by an acylamino group.

36. A compound as claimed in claim 30, wherein one of the radicals $R^2$ and $R^3$ represents H and the other I.

37. A compound as claimed in claim 30, wherein one of the

radicals $R^2$ and $R^3$ represents a hydrogen or halogen atom and the other represents an unsubstituted or substituted straight or branched chain aliphatic group.

38. A compound as claimed in claim 37, wherein an aliphatic group is a lower aliphatic group.

39. A compound as claimed in claim 37 or claim 38, wherein an aliphatic group $R^2$ or $R^3$ is substituted by an amino, mercapto, or hydroxyl group, which may itself be substituted.

40. A compound as claimed in claim 39, wherein $R^2$ and $R^3$ represents a hydroxymethyl, phenylhydroxymethyl, 1-hydroxyethyl or 2-hydroxy-2-propyl group.

41. A compound as claimed in claim 39, wherein an amino, mercapto or hydroxyl group is substituted by one of the following groups:

$-R^{10}$, $-CO-NR^{10}R''$, $-CO-R^{10}$,

$-CO-OR^{10}$, $-SO_2-R_{10}$, $-SO_2-OR^{10}$,

$-SO_3^{\ominus}$,

and in the case of an amino group $<\genfrac{}{}{0pt}{}{R^{10}}{R^{11}}$ in which groups $R^{10}$ and $R^{11}$, which may be the same or different, if appropriate, each represents an alkyl, aryl or aralkyl group, or a nitrogen atom present as a substituent of $R^2$ or $R^3$ or in the group $-CO-NR^{10}R^{11}$ may be present as part of an aromatic or non-aromatic heterocyclic ring.

42. A compound as claimed in claim 30, wherein R represents a carboxylic acid esterified with an aliphatic, cycloaliphatic, cycloaliphaticaliphatic aryl or araliphatic alcohol having up to 18 carbon atoms.

43. A compound of the general formula X

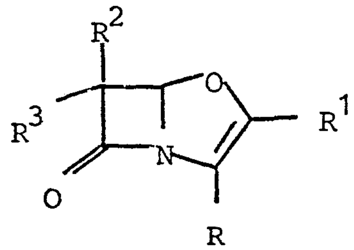

(X)

in which R, $R^1$, $R^2$ and $R^3$ are as defined in claim 1 for the general formula I, or as defined in any one of claims 31 to 42, with the exception of those compounds in which $R^2$ or $R^3$ represents an amino or substituted amino group directly bonded to the ring carbon atom, and those in which $R^2$ and $R^3$ both represent hydrogen atoms.

44. A compound of the general formula XI

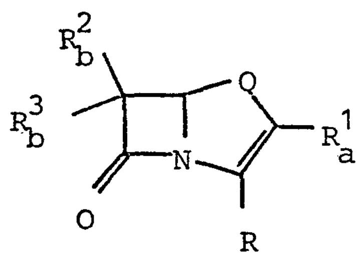

(XI)

in which $R_b^2$ and $R_b^3$ each represent a hydrogen atom, R is as defined in claim 1 for formula I and $R_a^1$ has the same meaning as $R^1$ in claim 1 or any one of claims 31 to 35, with the exception of vinyl groups, unsubstituted alkyl groups having up to 3 carbon atoms, methyl groups having one or two phenyl substituents and ethyl groups substituted by a hydroxyl, ether, or acyl, group or a S-containing nucleophilic group.

45. A compound of the general formula XII

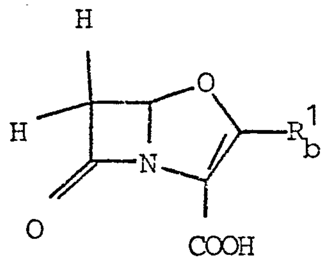

(XII)

in which $R_b^1$ represents an unsubstituted or substituted alkyl group having 3 or more carbon atoms, or a substituted methyl or substituted ethyl group.

46. A compound of the formula XIII

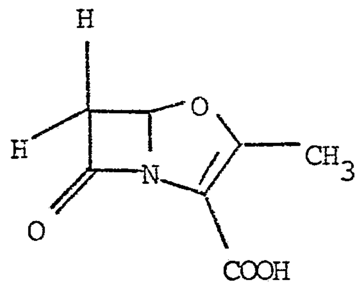

(XIII)

47. A compound as described in Table I herein.

48. A salt of a compound as claimed in any one of claims 30 to 47.

49. A physiologically tolerable salt of a compound as claimed in any one of claims 30 to 47.

50. A pharmaceutical preparation which comprises, as active ingredient, a compound as claimed in any one of claims 30 to 47, or a physiologically tolerable salt thereof, or a mixture of 2 or more such substances, in admixture or conjunction with a pharmaceutically suitable carrier.

51. A pharmaceutical preparation as claimed in claim 50,

which also comprises one or more other pharmaceutically active substances.

52. A pharmaceutical preparation as claimed in claim 51, wherein the pharmaceutically active substance has antibacterial activity.

53. A pharmaceutical preparation as claimed in claim 52, wherein the antibacterial substance has a ß-lactam ring.

54. A pharmaceutical preparation as claimed in any one of claims 50 to 53, in unit dosage form.

55. A pharmaceutical preparation which comprises a compound as claimed in any one of claims 30 to 47, or a physiologically tolerable salt thereof, in unit dosage form.

56. A pharmaceutical preparation as claimed in claim 55, which also comprises one or more other pharmaceutically active substances.

57. A pharmaceutical preparation as claimed in claim 56, wherein the pharmaceutically active substance has antibacterial activity.

58. A pharmaceutical preparation as claimed in claim 57, wherein the antibacterial substance has a ß-lactam ring.

59. The use of a compound as claimed in any one of claims 30 to 47 or a physiologically tolerable salt thereof, as a ß-lactamase inhibitor and/or as an antibacterial agent.

60. A compound of formula II

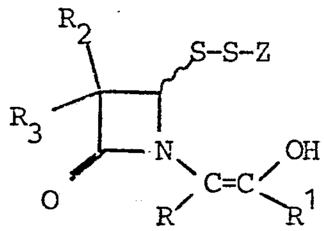

(II)

in which R, $R^1$, $R^2$, $R^3$ and Z are as defined in any one of claims 1, 24 to 27 and 31 to 42.

61. A compound of formula IIa

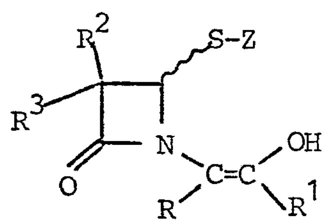

(IIa)

in which R, $R^1$, $R^2$, $R^3$ and Z are as defined in any one of claims 1, 24 to 27 and 31 to 42.

62. A compound as claimed in claim 60 or 61, wherein one of the radicals $R^2$ and $R^3$ represents a hydrogen atom and the other represents an iodine atom, or a hydroxy-substituted lower aliphatic group which may itself be substituted.

63. A compound as claimed in claim 62, wherein a hydroxy-substituted lower aliphatic group is a hydroxymethyl, phenylhydroxymethyl, 1-hydroxyethyl or 2-hydroxy-2-propyl group.

64. A compound of formula IIIa

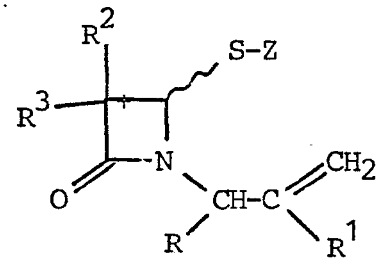

(IIIa)

in which R, $R^1$, $R^2$, $R^3$ and Z are as defined in any one of claims 1, 24 to 27 and 31 to 42.

65. A compound of formula III

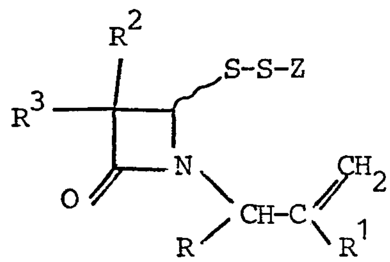

(III)

in which R, $R^1$, $R^2$, $R^3$ and Z are as defined in any one of claims 1, 24 to 27 and 31 to 42 with the proviso that $R^2$ and $R^3$ may not both represent hydrogen atoms.

66. A compound of formula IVa

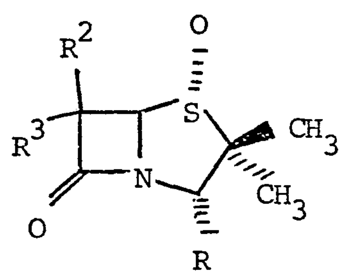

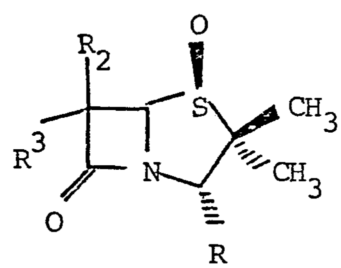

(IVa)

in which R, $R^2$ and $R^3$ are as defined in any one of claims 1 and 37 to 42, with the proviso that $R^2$ and $R^3$ may not both represent hydrogen atoms.

67. A compound as claimed in claim 66, wherein one of the radicals $R^2$ and $R^3$ represents a hydrogen atom and the other represents an iodine atom.

68. A compound as claimed in claim 66, wherein one of the radicals $R^2$ and $R^3$ represents a hydrogen atom and the other represents a hydroxy-substituted lower aliphatic group, which may itself be substituted.

69. A compound as claimed in claim 68, wherein a hydroxy-substituted lower aliphatic group is a hydroxymethyl, phenylhydroxymethyl, 1-hydroxyethyl, or 2-hydroxy-2-propyl group.

70. A compound of formula VI

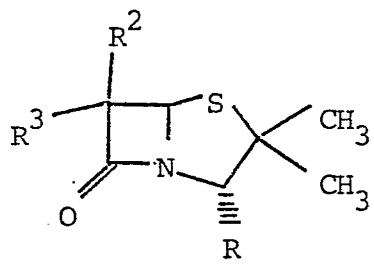

(VI)

in which R, $R^2$ and $R^3$ are as defined in any one of claims 1 and 37 to 42, with the proviso that $R^2$ and $R^3$ may not both represent hydrogen atoms.

71. A compound as claimed in claim 70, wherein one of the radicals $R^2$ and $R^3$ represents an iodine atom and the other represents a hydrogen atom, with the exception of those compounds in which R represents a COOH, $-COOH_3$ or $-COOCH_2C_6H_5$ group.

72. A compound as claimed in claim 70, wherein $R^2$ and $R^3$ are as defined in claim 68 or 69.

73. A compound of formula IVb

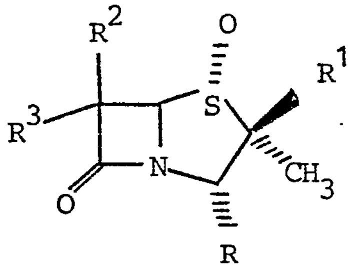

(IVb)

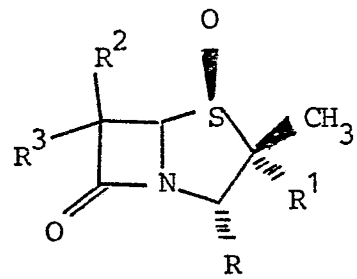

in which R, $R^1$, $R^2$, $R^3$ are as defined in any one of claims 1 and 31 to 42.

74. A compound as claimed in claim 73, wherein $R^2$ and $R^3$ are as defined in claim 67, claim 68 or claim 69.

75. A compound of formula IXa

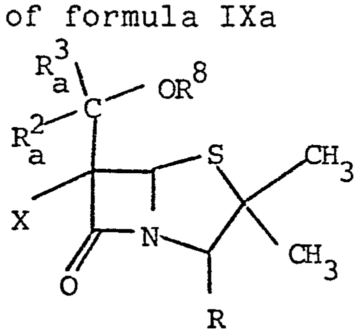

(IXa)

wherein $R_a^2$ and $R_a^3$ are as defined for $R^2$ and $R^3$ in any one of claims 1 and 37 to 41, and may also represent aryl, and $R^8$ with the proviso that the group at position 6 may not represent a 1-hydroxyethyl group.

76. A compound of formula IXc

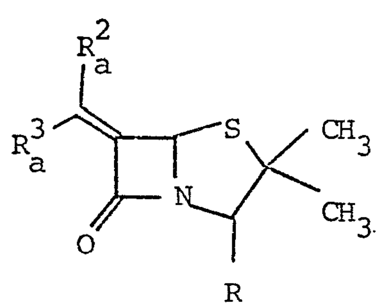

(IXc)

0018305
HOE 79/F 346

in which R is as defined in claim 1 or claim 42 and $R_a^2$ and $R_a^3$ are as defined for $R^2$ and $R^3$ in any one of claims 1 and 37 to 41, with the proviso that one of $R_a^2$ and $R_a^3$ may not represent a methyl group when the other represents a hydrogen atom.

European Patent Office

# EUROPEAN SEARCH REPORT

0018305

Application number

EP 80710008.6

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| | DE - A1 - 2 740 526 (GLAXO)<br>+ Pages 1-10,17 + | 1-6, 30-49 | C 07 D 498/04<br>C 07 D 205/08<br>C 07 D 499/00<br>A 61 K 31/42//<br>(C 07 D 498/04,<br>C 07 D 205/00,<br>C 07 D 263/00) |
| | DE - A1 - 2 633 561 (BEECHAM)<br>+ Pages 70-75 + | 1-6, 30-59 | |
| | AT - B - 331 399 (GIST-BROCADES)<br>+ Pages 18-22 + | 7-13, 64,66, 73 | |
| | AT - B - 327 389 (FUJISAWA)<br>+ Totality + | 7-13,65, 66,73 | TECHNICAL FIELDS SEARCHED (Int.Cl. 3) |
| | AT - B - 316 741 (ELI LILLY)<br>+ Pages 7,8 + | 7-13, 64,66, 73 | C 07 D 498/00<br>C 07 D 205/00<br>C 07 D 499/00<br>A 61 K 31/00 |
| | US - A - 4 123 539 (DI NINNO)<br>+ Totality + | 66,68-70,72-75 | |
| P | EP - A1 - 0 005 889 (SCHERICO) (12-12-1979)<br>+ Pages 63-70 + | 14-16, 30,37-40,66-75 | |
| | AT - B - 329 757 (FUJISAWA)<br>+ Page 7 + | 14,66, 70 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | AT - B - 307 619 (ELI LILLY)<br>+ Pages 8,9 + | 14,66, 70 | |
| | GB - A - 2 000 138 (PFIZER) (04-01-1979)<br>+ Pages 11,12,15,16 + | 66,70, 73 | &: member of the same patent family, corresponding document |

X The present search report has been drawn up for all claims

| Place of search | Date of completion of the search | Examiner |
|---|---|---|
| VIENNA | 20-06-1980 | JANISCH |

EPO Form 1503.1 06.78